# EUROPEAN PATENT SPECIFICATION

(11) **EP 2 762 485 B1**
(45) Date of publication and mention of the grant of the patent: **01.08.2018**
(21) Application number: 12837234.9
(22) Date of filing: 21.09.2012
(51) Int. Cl.: C07K 1/06, C07K 1/12, C12P 21/00, C07C 279/22, C12N 15/00

(54) **METHOD FOR PRODUCING POLYPEPTIDE FRAGMENT WITH HIGH EFFICIENCY, WHICH IS SUITABLE FOR NCL METHOD**
VERFAHREN ZUR HERSTELLUNG VON POLYPEPTIDFRAGMENTEN MIT HOHER WIRKUNG FÜR NCL-VERFAHREN
PROCÉDÉ D'OBTENTION DE FRAGMENT POLYPEPTIDIQUE DE RENDEMENT ÉLEVÉ, QUI EST APPROPRIÉ POUR UN PROCÉDÉ NCL

(30) Priority: 26.09.2011 JP 2011210007
(43) Date of publication of application: 06.08.2014
(73) Proprietor: Glytech, Inc., Kyoto 600-8813 (JP)
(72) Inventor: KAJIHARA, Yasuhiro, Toyonaka-shi Osaka 560-0043 (JP); OKAMOTO, Ryo, Toyonaka-shi Osaka 560-0043 (JP); KIMURA, Motoharu, Toyonaka-shi Osaka 560-0043 (JP); ISHII, Kazuyuki, Kyoto-shi Kyoto 600-8813 (JP)
(74) Representative: J A Kemp
(86) International application number: PCT/JP2012/074239
(87) International publication number: WO 2013/047372

(56) References cited:
- WO-A1-2010/092943
- WO-A1-2010/150730
- JP-A- 2009 242 372
- RICHARDSON JONATHAN P ET AL: "Optimisation of chemical protein cleavage for erythropoietin semi-synthesis using native chemical ligation", ORGANIC & BIOMOLECULAR CHEMISTRY, ROYAL SOCIETY OF CHEMISTRY, GB , vol. 6, no. 21 7 November 2008 (2008-11-07), pages 3977-3982, XP002684661, ISSN: 1477-0520, DOI: 10.1039/B811501J Retrieved from the Internet: URL:http://pubs.rsc.org/en/Content/Article Landing/2008/OB/b811501j [retrieved on 2008-09-05]
- YASUHIRO KAJIWARA ET AL.: 'Chemical synthesis of glycoproteins having complex type oligosaccharides' BULLETIN OF YOKOHAMA CITY UNIVERSITY, NATURAL SCIENCE vol. 60, no. 1, 2010, pages 57 - 69, XP008173629
- JOHNSON, E. C. B. ET AL.: 'Insights into the Mechanism and Catalysis of the Native Chemical Ligation Reaction.' J. AM. CHEM. SOC. vol. 128, 2006, pages 6640 - 6646, XP008149115

### Sequence Listing

### OCKP1105F sequence listing.txt

### SEQUENCE LISTING

<110> GLYTECH, INC.
<120> Method for producing polypeptide fragment available for Native
   Chemical Ligation
<130> OCKP1105F
<150> JP2011-210007
   <151> 2011-09-26
<160> 21
<170> PatentIn version 3.5.1
<210> 1
   <211> 112
   <212> PTR
   <213> Homo sapiens
<220>
   <221> CARBOHYD
   <222> (52)..(52)
   <223> asialo oligosaccharide
<400> 1
   Gly Pro Val Pro Pro Ser Thr Ala Leu Arg Glu Leu Ile Glu Glu Leu
   1               5                   10                  15
   Val Asn Ile Thr Gln Asn Gln Lys Ala Pro Leu Cys Asn Gly Ser Met
               20                  25                  30
   Val Trp Ser Ile Asn Leu Thr Ala Gly Met Tyr Cys Ala Ala Leu Glu
           35                  40                  45
   Ser Leu Ile Asn Val Ser Gly Cys Ser Ala Ile Glu Lys Thr Gln Arg
       50                  55                  60
   Ala Leu Ser Gly Phe Cys Pro His Lys Val Ser Ala Gly Gln Phe Ser
   65                  70                  75                      80
   Ser Leu His Val Arg Asp Thr Lys Ile Glu Val Ala Gln Phe Val Lys
                   85                  90                  95
   Asp Leu Leu Leu His Leu Lys Lys Leu Phe Arg Glu Gly Arg Phe Asn
               100                 105                 110
<210> 2
   <211> 27
   <212> PRT
   <213> Artificial
<220>
   <223> chemically synthesized
<400> 2
   Gly Pro Val Pro Pro Ser Thr Ala Leu Arg Glu Leu Ile Glu Glu Leu
   1               5                   10                  15
   Val Asn Ile Thr Gln Asn Gln Lys Ala Pro Leu
               20                  25
<210> 3
   <211> 57
   <212> PRT
   <213> Artificial
<220>
   <223> chemically synthesized
<400> 3
   Cys Ser Ala Ile Glu Lys Thr Gln Arg Ala Leu Ser Gly Phe Cys Pro
   1 5 10 15
   His Lys Val Ser Ala Gly Gln Phe Ser Ser Leu His Val Arg Asp Thr
   20 25 30
   Lys Ile Glu Val Ala Gln Phe Val Lys Asp Leu Leu Leu His Leu Lys
   35 40 45
   Lys Leu Phe Arg Glu Gly Arg Phe Asn
   50 55
<210> 4
   <211> 253
   <212> PRT
   <213> Artificial
<220>
   <223> chemically synthesized
<400> 4
   Met Ser Asp Lys Ile Ile His Leu Thr Asp Asp Ser Phe Asp Thr Asp
   1 5 10 15
   Val Leu Lys Ala Asp Gly Ala Ile Leu Val Asp Phe Trp Ala Glu Trp
   20 25 30
   Cys Gly Pro Cys Lys Met Ile Ala Pro Ile Leu Asp Glu Ile Ala Asp
   35 40 45
   Glu Tyr Gln Gly Lys Leu Thr Val Ala Lys Leu Asn Ile Asp Gln Asn
   50 55 60
   Pro Gly Thr Ala Pro Lys Tyr Gly Ile Arg Gly Ile Pro Thr Leu Leu
   65 70 75 80
   Leu Phe Lys Asn Gly Glu Val Ala Ala Thr Lys Val Gly Ala Leu Ser
                   85                  90                  95
   Lys Gly Gln Leu Lys Glu Phe Leu Asp Ala Asn Leu Ala Gly Ser Gly
               100                 105                 110
   Ser Gly His Met His His His His His His Ser Ser Gly Leu Val Pro
           115                 120                 125
   Arg Gly Ser Gly Met Lys Glu Thr Ala Ala Ala Lys Phe Glu Arg Gln
       130                 135                 140
   His Met Asp Ser Pro Asp Leu Gly Thr Asp Asp Asp Asp Lys Ala Met
   145                 150                 155                 160
   Gly Pro Val Pro Pro Ser Thr Ala Leu Arg Glu Leu Ile Glu Glu Leu
                   165                 170                 175
   Val Asn Ile Thr Gln Asn Gln Lys Ala Pro Leu Cys Val His His His
               180                 185                 190
   His His His Met Cys Ser Ala Ile Glu Lys Thr Gln Arg Ala Leu Ser
           195                 200                 205
   Gly Phe Cys Pro His Lys Val Ser Ala Gly Gln Phe Ser Ser Leu His
       210                 215                 220
   Val Arg Asp Thr Lys Ile Glu Val Ala Gln Phe Val Lys Asp Leu Leu
   225                 230                 235                 240
   Leu His Leu Lys Lys Leu Phe Arg Glu Gly Arg Phe Asn
                   245                 250
<210> 5
   <211> 282
   <212> DNA
   <213> Artificial
<220>
   <223> chemically synthesized
<400> 5
   atgggtccgg ttccgcctag taccgcactg cgtgagctga ttgaagaact ggtcaacatt     60
   acccagaacc aaaaggcccc gctgtgcgta catcaccacc atcatcacat gtgctctgcg    120
   atcgaaaaga cgcaacgcgc gctgagtggc ttctgtccac acaaggtgag cgccggtcag    180
   ttctctagcc tgcacgttcg tgacactaaa atcgaagtgg ctcagtttgt gaaagacctg    240
   ctgctgcacctgaagaaactgtttcgtgagggccgctttaat                           282
<210> 6
   <211> 36
   <212> PRT
   <213> Artificial
<220>
   <223> chemically synthesized
<220>
   <221> MISC_FEATURE
   <222> (36)..(36)
   <223> X is HomoSerine Lactone
<400> 6
   Gly Pro Val Pro Pro Ser Thr Ala Leu Arg Glu Leu Ile Glu Glu Leu
   1               5                   10                  15
   Val Asn Ile Thr Gln Asn Gln Lys Ala Pro Leu Cys Val His His His
               20                  25                  30
   His His His Xaa
           35
<210> 7
   <211> 37
   <212> PRT
   <213> Artificial
<220>
   <223> chemically synthesized
<220>
   <221> BINDING
   <222> (1)..(1)
   <223> Boc
<220>
   <221> BINDING
   <222> (24)..(24)
   <223> Boc
<220>
   <221> MISC_FEATURE
   <222> (36)..(36)
   <223> X is HomoSerine Lactone
<400> 7
<210> 8
   <211> 36
   <212> PRT
   <213> Artificial
<220>
   <223> chemically synthesized
<220>
   <221> BINDING
   <222> (1)..(1)
   <223> Boc
<220>
   <221> BINDING
   <222> (24)..(24)
   <223> Boc
<220>
   <221> BINDING
   <222> (28)..(28)
   <223> C(=S)OPh
<220>
   <221> MISC_FEATURE
   <222> (36)..(36)
   <223> X is HomoSerine Lactone
<400> 8
   Gly Pro Val Pro Pro Ser Thr Ala Leu Arg Glu Leu Ile Glu Glu Leu
   1               5                   10                  15
   Val Asn Ile Thr Gln Asn Gln Lys Ala Pro Leu Cys Val His His His
               20                  25                  30
   His His His Xaa
           35
<210> 9
   <211> 27
   <212> PRT
   <213> Artificial
<220>
   <223> chemically synthesized
<220>
   <221> BINDING
   <222> (1)..(1)
   <223> Boc
<220>
   <221> BINDING
   <222> (24)..(24)
   <223> Boc
<220>
   <221> BINDING
   <222> (27)..(27)
   <223> -NH-C=NH(NHAc)
<400> 9
   Gly Pro Val Pro Pro Ser Thr Ala Leu Arg Glu Leu Ile Glu Glu Leu
   1               5                   10                  15
   Val Asn Ile Thr Gln Asn Gln Lys Ala Pro Leu
               20                  25
<210> 10
   <211> 16
   <212> PRT
   <213> Artificial
<220>
   <223> chemically synthesized
<220>
   <221> MISC_FEATURE
   <222> (1)..(1)
   <223> 1C has Thiazolidine gourp
<220>
   <221> BINDING
   <222> (16)..(16)
   <223> -S-CH2CH2CONHCH(CH2CH(CH3)2)COOH
<400> 10
   Cys Asn Gly Ser Met Val Trp Ser Ile Asn Leu Thr Ala Gly Met Tyr
   1               5                   10                  15
<210> 11
   <211> 12
   <212> PRT
   <213> Artificial
<220>
   <223> chemically synthesized
<220>
   <221> MISC_FEATURE
   <222> (1)..(1)
   <223> 1C has Thiazolidine gourp
<220>
   <221> BINDING
   <222> (1)..(1)
   <223> Boc
<220>
   <221> BINDING
   <222> (5)..(5)
   <223> OtBu
<220>
   <221> BINDING
   <222> (6)..(6)
   <223> tBu
<220>
   <221> BINDING
   <222> (11)..(11)
   <223> tBu
<220>
   <221> CARBOHYD
   <222> (9)..(9)
   <223> asialo oligosaccharide
<400> 11
   Cys Ala Ala Leu Glu Ser Leu Ile Asn Val Ser Gly
   1               5                   10
<210> 12
   <211> 12
   <212> PRT
   <213> Artificial
<220>
   <223> chemically synthesized
<220>
   <221> MISC_FEATURE
   <222> (1)..(1)
   <223> 1C has Thiazolidine gourp
<220>
   <221> CARBOHYD
   <222> (9)..(9)
   <223> asialo oligosaccharide
<400> 12
   Cys Ala Ala Leu Glu Ser Leu Ile Asn Val Ser Gly
   1               5                   10
<210> 13
   <211> 12
   <212> PRT
   <213> Artificial
<220>
   <223> chemically synthesized
<220>
   <221> MISC_FEATURE
   <222> (1)..(1)
   <223> 1C has Thiazolidine gourp
<220>
   <221> BINDING
   <222> (1)..(1)
   <223> Boc
<220>
   <221> BINDING
   <222> (5)..(5)
   <223> OtBu
<220>
   <221> BINDING
   <222> (6)..(6)
   <223> tBu
<220>
   <221> BINDING
   <222> (11)..(11)
   <223> tBu
<220>
   <221> BINDING
   <222> (12)..(12)
   <223> -SBn
<220>
   <221> CARBOHYD
   <222> (9)..(9)
   <223> asialo oligosaccharide
<400> 13
   Cys Ala Ala Leu Glu Ser Leu Ile Asn Val Ser Gly
   1               5                   10
<210> 14
   <211> 12
   <212> PRT
   <213> Artificial
<220>
   <223> chemically synthesized
<220>
   <221> MISC_FEATURE
   <222> (1)..(1)
   <223> 1C has Thiazolidine gourp
<220>
   <221> BINDING
   <222> (12)..(12)
   <223> -SBn
<220>
   <221> CARBOHYD
   <222> (9)..(9)
   <223> asialo oligosaccharide
<400> 14
   Cys Ala Ala Leu Glu Ser Leu Ile Asn Val Ser Gly
   1               5                   10
<210> 15
   <211> 69
   <212> PRT
   <213> Artificial
<220>
   <223> chemically synthesized
<220>
   <221> MISC_FEATURE
   <222> (1)..(1)
   <223> 1C has Thiazolidine gourp
<220>
   <221> CARBOHYD
   <222> (9)..(9)
   <223> asialo oligosaccharide
<400> 15
   Cys Ala Ala Leu Glu Ser Leu Ile Asn Val Ser Gly Cys Ser Ala Ile
   1               5                   10                  15
   Glu Lys Thr Gln Arg Ala Leu Ser Gly Phe Cys Pro His Lys Val Ser
               20                  25                  30
   Ala Gly Gln Phe Ser Ser Leu His Val Arg Asp Thr Lys Ile Glu Val
           35                  40                  45
   Ala Gln Phe Val Lys Asp Leu Leu Leu His Leu Lys Lys Leu Phe Arg
       50                  55                  60
   Glu Gly Arg Phe Asn
   65
<210> 16
   <211> 69
   <212> PRT
   <213> Artificial
<220>
   <223> chemically synthesized
<220>
   <221> CARBOHYD
   <222> (9)..(9)
   <223> asialo oligosaccharide
<400> 16
   Cys Ala Ala Leu Glu Ser Leu Ile Asn Val Ser Gly Cys Ser Ala Ile
   1               5                   10                  15
   Glu Lys Thr Gln Arg Ala Leu Ser Gly Phe Cys Pro His Lys Val Ser
               20                  25                  30
   Ala Gly Gln Phe Ser Ser Leu His Val Arg Asp Thr Lys Ile Glu Val
           35                  40                  45
   Ala Gln Phe Val Lys Asp Leu Leu Leu His Leu Lys Lys Leu Phe Arg
       50                  55                  60
   Glu Gly Arg Phe Asn
   65
<210> 17
   <211> 85
   <212> PRT
   <213> Artificial
<220>
   <223> chemically synthesized
<220>
   <221> MISC_FEATURE
   <222> (1)..(1)
   <223> 1C has Thiazolidine gourp
<220>
   <221> CARBOHYD
   <222> (25)..(25)
   <223> asialo oligosaccharide
<400> 17
   Cys Asn Gly Ser Met Val Trp Ser Ile Asn Leu Thr Ala Gly Met Tyr
   1 5 10 15
   Cys Ala Ala Leu Glu Ser Leu Ile Asn Val Ser Gly Cys Ser Ala Ile
   20 25 30
   Glu Lys Thr Gln Arg Ala Leu Ser Gly Phe Cys Pro His Lys Val Ser
   35 40 45
   Ala Gly Gln Phe Ser Ser Leu His Val Arg Asp Thr Lys Ile Glu Val
   50 55 60
   Ala Gln Phe Val Lys Asp Leu Leu Leu His Leu Lys Lys Leu Phe Arg
   65 70 75 80
   Glu Gly Arg Phe Asn
   85
<210> 18
   <211> 85
   <212> PRT
   <213> Artificial
<220>
   <223> chemically synthesized
<220>
   <221> CARBOHYD
   <222> (25)..(25)
   <223> asialo oligosaccharide
<400> 18
   Cys Asn Gly Ser Met Val Trp Ser Ile Asn Leu Thr Ala Gly Met Tyr
   1 5 10 15
   Cys Ala Ala Leu Glu Ser Leu Ile Asn Val Ser Gly Cys Ser Ala Ile
   20 25 30
   Glu Lys Thr Gln Arg Ala Leu Ser Gly Phe Cys Pro His Lys Val Ser
   35 40 45
   Ala Gly Gln Phe Ser Ser Leu His Val Arg Asp Thr Lys Ile Glu Val
   50 55 60
   Ala Gln Phe Val Lys Asp Leu Leu Leu His Leu Lys Lys Leu Phe Arg
   65 70 75 80
   Glu Gly Arg Phe Asn
   85
<210> 19
   <211> 112
   <212> PRT
   <213> Artificial
<220>
   <223> chemically synthesized
<220>
   <221> BINDING
   <222> (1)..(1)
   <223> Boc
<220>
   <221> BINDING
   <222> (24)..(24)
   <223> Boc
<220>
   <221> CARBOHYD
   <222> (52)..(52)
   <223> asialo oligosaccharide
<400> 19
   Gly Pro Val Pro Pro Ser Thr Ala Leu Arg Glu Leu Ile Glu Glu Leu
   1               5                   10                  15
   Val Asn Ile Thr Gln Asn Gln Lys Ala Pro Leu Cys Asn Gly Ser Met
               20                  25                  30
   Val Trp Ser Ile Asn Leu Thr Ala Gly Met Tyr Cys Ala Ala Leu Glu
           35                  40                  45
   Ser Leu Ile Asn Val Ser Gly Cys Ser Ala Ile Glu Lys Thr Gln Arg
       50                  55                  60
   Ala Leu Ser Gly Phe Cys Pro His Lys Val Ser Ala Gly Gln Phe Ser
   65                  70                  75                  80
   Ser Leu His Val Arg Asp Thr Lys Ile Glu Val Ala Gln Phe Val Lys
                   85                  90                  95
   Asp Leu Leu Leu His Leu Lys Lys Leu Phe Arg Glu Gly Arg Phe Asn
               100                 105                 110
<210> 20
   <211> 13
   <212> PRT
   <213> Artificial
<220>
   <223> chemically synthesized
<220>
   <221> BINDING
   <222> (1)..(1)
   <223> Boc
<220>
   <221> BINDING
   <222> (6)..(6)
   <223> C(=S)OPh
<220>
   <221> MISC_FEATURE
   <222> (7)..(7)
   <223> X is G, V, I, P, F, W, or L
<400> 20
   Asp Val Ala Asp Phe Cys Xaa His His His His His His
   1               5                   10
   13
<210> 21
   <211> 5
   <212> PRT
   <213> Artificial
<220>
   <223> chemically synthesized
<220>
   <221> BINDING
   <222> (1)..(1)
   <223> Boc
<220>
   <221> BINDING
   <222> (5)..(5)
   <223> -NH-C=NH(NHAc)
<400> 21
   Asp Val Ala Asp Phe
   1               5

## Description

### Technical Field

The present invention relates to a method for efficiently manufacturing a polypeptide fragment suitable for the NCL method.

### Background Art

Various methods such as biosynthesis, chemical synthesis, and cell-free synthesis are known for protein synthesis. Cells such as *E. coli* are utilized in a biosynthesis method, and DNA encoding the protein to be synthesized are introduced into a cell and expressed to give the protein. In a chemical synthesis, the protein of interest is synthesized by binding amino acids in order by organic chemistry. Moreover, in a cell-free synthesis, the protein is synthesized without a cell by utilizing enzymes etc. present in various cells such as *E. coli.* These methods can be selected or used in combination as appropriate depending on e.g. the intended use or the size of the protein, or the property rendered.

In order to synthesize a protein uniformly having a particular modification such as a sugar chain or a lipid in the mid-portion of the amino acid sequence, a method for individually synthesizing a peptide fragment comprising a sugar chain and a peptide fragment that does not comprise a sugar chain, and then synthesizing the final protein by ligation is employed. The peptide fragment comprising a sugar chain can be synthesized by a chemical synthesis employing an amino acid modified with a sugar chain or a lipid etc. in advance. Moreover, the synthesis of the peptide fragment portion that does not comprise a sugar chain can be manufactured by chemical synthesis or biosynthesis.

Solid phase synthesis method is mainly employed as the method for chemically synthesizing a peptide chain. However, a peptide chain obtained by the solid phase synthesis method is generally a short chain, approximately 50 residues at the longest. It is therefore preferred to employ biosynthesis for synthesizing a long peptide chain.

Various methods are reported as methods for linking peptide chains, and one method that is broadly employed is the Native Chemical Ligation method (NCL method). The NCL method can also be performed between unprotected peptide chains, and is known to be a method useful for producing a natural amide bond (peptide bond) at the linking site (ligation site) (such as Patent Literature 1). The NCL method is a chemical selective reaction between a first peptide made to have an α carboxythioester moiety at the C-terminal and a second peptide having a cysteine residue at the N-terminal, wherein the thiol group on the side chain of the cysteine (SH group, may be referred to as a sulfhydryl group) selectively reacts with the carbonyl carbon of the thioester group and a thioester bond early intermediate is produced by a thiol exchange reaction. This intermediate is spontaneously intramolecularly rearranged, rendering a natural amide bond to the linking site, while regenerating the cysteine side chain thiol.

This method is a method that can link two peptide chains via a peptide bond merely by employing unprotected peptides and mixing them in a buffer solution. The NCL method can selectively link the C-terminal of one peptide with the N-terminal of the other peptide, even in the case of a reaction between compounds having numerous functional groups such as a peptide. Accordingly, the way how the NCL method is utilized will be important in chemically synthesizing a protein.

However, a problem in utilizing the NCL method includes the preparation of a peptide thioester form having an α carboxythioester moiety at the C-terminal which is necessary as the source material. Although various methods are reported as methods for preparing a peptide thioester (such as Non-Patent Literature 1 and Patent Literature 2), all methods have their basis in solid phase synthesis and are therefore constrained by the restrictions thereof, and the size of the peptide thioester form that can be synthesized is restricted. Further, in a method employing a linker, a non-natural amino acid derivative or a special derivative must be separately chemically synthesized, and it cannot be said that the procedures therefor are always easy.

Meanwhile, while a method for obtaining a polypeptide fragment biosynthesized by a cell as a thioester form (Intein method) has also been reported (Non-Patent Literature 2). When employing this method, however, a target peptide sequence will be necessary not only to express the polypeptide but also to allow functioning of protein splicing, and the expressed intein-complex protein must always be folded and take the intrinsic three-dimensional structure. For this reason, the peptide thioester is not always obtained depending on the polypeptide sequence to be expressed, the optimization of the sufficient conditions are always involved, and complexity of operation is associated.

Moreover, separately from the Intein method, a method for manufacturing a thioester form that can be applied to a biosynthesized polypeptide is also reported (Patent Literature 3). This method is a method for activating a cysteine comprised in the synthesized polypeptide to thereby cleave off the thioester form at the activated cysteine position. In other words, the peptide fragment on the N-terminal side of the activated cysteine can be obtained as a thioester form. However, with this method, the polypeptide cleaved off on the C-terminal side of activated cysteine forms a chemically stable cyclic structure at its N-terminal when cleaved off, and thus could not be used in any later protein synthesis. For this reason, there was a need to separately manufacture the peptide fragment on the N-terminal side as a ligatable peptide fragment.

Moreover, in biosynthesis, although full length proteins are properly expressed, peptide fragments are sometimes not properly expressed, for example due to being recognized as a mistake in a cell and degraded. Accordingly, when the necessary peptide fragment is cleaved off as a thioester form with the above method after biosynthesizing a full length protein, the long chain polypeptide on the C-terminal side of the peptide fragment cleaved off could not be utilized.

### Citation List

### [Patent Literatures]

[Patent Literature 1] International Publication No. 96/34878
[Patent Literature 2] International Publication No. 2007/114454
[Patent Literature 3] International Publication No. 2010/150730

### [Non-Patent Literatures]

[Non-Patent Literature 1] Ingenito et al., J. Am. Chem. Soc. 1999, 121, 11369-11374
[Non-Patent Literature 2] Schwartz et al., CHEM. COMMUN., 2003 2087-2090

### Summary of the Invention

### Problems to be Solved by the Invention

The object of the present invention is to provide a method for efficiently manufacturing a first polypeptide fragment having cysteine at the N-terminal and a second polypeptide fragment having the C-terminal modified which are suitable for the NCL method.

### Means for Solving the Problems

As a result of repeated research to solve the above problems, the present inventors found that a first polypeptide fragment having cysteine at the N-terminal and a second polypeptide fragment having the C-terminal modified which are suitable for the NCL method can be efficiently manufactured by preparing a first polypeptide fragment and a second polypeptide fragment as one polypeptide intervened by a particular sequence.

In other words, the present invention relates to a method for efficiently manufacturing a first polypeptide fragment having cysteine at the N-terminal and a second polypeptide fragment having the C-terminal modified which are suitable for the NCL method, comprising:
(1) A step of reacting a polypeptide having the following structure:
   (N-terminal) second polypeptide fragment-Cys-W-(His)n-Z-Met-first polypeptide fragment (C-terminal)
   [wherein n means an integer from 0 - 10, Cys is cysteine, W means any 1, 2, or 3 amino acids, Z means any 0, 1, or 2 amino acids, His means histidine, Met means methionine, and the N-terminal of the first polypeptide fragment is cysteine]
   with CNBr to obtain the following polypeptide fragments:
   (A) a first polypeptide fragment having cysteine at the N-terminal and
   (B) a third polypeptide fragment having the following structure:
      (N-terminal) second polypeptide fragment-Cys-W-(His)n-Z-Met' (C-terminal) [wherein Met' means a Met derivative]; and
(2) A step of reacting said third polypeptide fragment with a compound represented by the following formula (I): 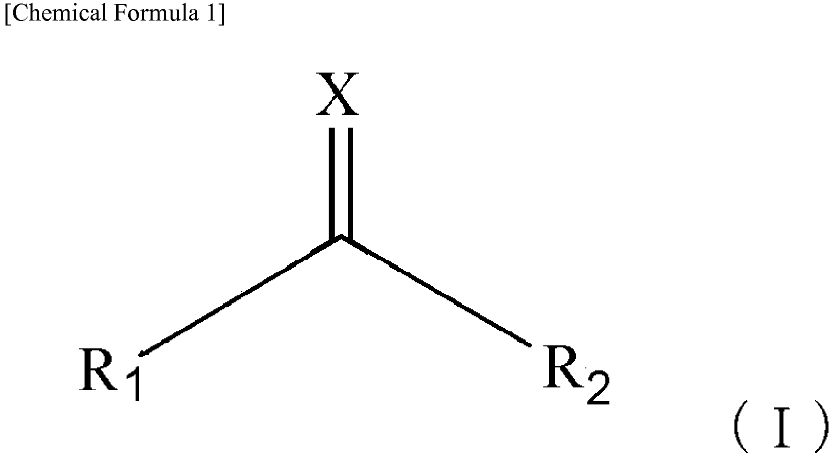 [wherein
   X is a sulfur atom or an oxygen atom, and
   R₁ and R₂ are leaving groups]
   and subsequently, in an organic solvent, reacting with a compound represented by the following formula (II): 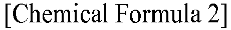 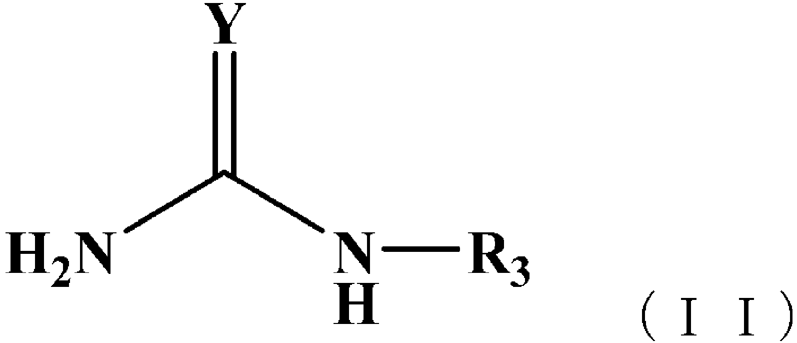 [wherein
   Y is an oxygen atom, a sulfur atom, or =NH, and
   R₃ is a hydrogen atom, an acyl group, or an alkoxycarbonyl group] to obtain a second polypeptide fragment having the C-terminal modified having the following structure:
      (N-terminal) second polypeptide fragment-C(=O)-NH-C(=Y)NHR₃ (C-terminal).

Here, one embodiment of the manufacturing method of the present invention is characterized in that it further comprises a step of reacting the second polypeptide fragment having the C-terminal modified obtained in said step (2) with further a thiol represented by the following formula:

R₄-SH

[wherein R₄ is any one group selected from the group consisting of a substituted or unsubstituted benzyl group, a substituted or unsubstituted aryl group, and a substituted or unsubstituted alkyl group]
and exchanging the -NH-C(=Y)NHR₃ group at the C-terminal with the thiol group to obtain a second polypeptide fragment having the C-terminal modified having the following structure:
(N-terminal) second polypeptide fragment-C(=O)-SR₄ (C-terminal).

Moreover, one embodiment of the manufacturing method of the present invention is characterized in that X in the compound represented by said formula (I) is a sulfur atom.

Moreover, one embodiment of the manufacturing method of the present invention is characterized in that R₁ in the compound represented by said formula (I) is an -O-C₆ aryl group.

Moreover, one embodiment of the manufacturing method of the present invention is characterized in that R₂ in the compound represented by said formula (I) is a halogen atom or a substituted or unsubstituted -S-C₆₋₁₀ aryl group.

Moreover, one embodiment of the manufacturing method of the present invention is characterized in that Y in the compound represented by said formula (II) is =NH.

Moreover, one embodiment of the manufacturing method of the present invention is characterized in that R₃ in the compound represented by said formula (II) is an acetyl group.

Moreover, one embodiment of the manufacturing method of the present invention is characterized in that said polypeptide having the following structure:
(N-terminal) second polypeptide fragment-Cys-W-(His)n-Z-Met-first polypeptide fragment (C-terminal)
is a recombinant polypeptide fragment expressed by a cell.

Moreover, one embodiment of the manufacturing method of the present invention is characterized in that said cell is *E. coli.*

Moreover, one embodiment of the manufacturing method of the present invention is characterized in that W is one amino acid, and is any one amino acid selected from the group consisting of Val, Ile, Leu, and Trp.

Moreover, one embodiment of the manufacturing method of the present invention is characterized in that n is 6 - 10.

Moreover, another aspect of the present invention relates to a method for manufacturing a first polypeptide fragment having cysteine at the N-terminal suitable for the NCL method, characterized in that a polypeptide having the following structure:
(N-terminal) second polypeptide fragment-P-Met-first polypeptide fragment (C-terminal)
[wherein P is any 0 - 10 amino acids, Met means methionine, and the N-terminal of the first polypeptide fragment is cysteine]
is reacted with CNBr.

Moreover, another aspect of the present invention relates to a method for manufacturing a glycosylated polypeptide, comprising:
(1) A step of classifying and designing the peptide sequence of the desired glycosylated polypeptide to be manufactured into at least:
   - a sugar chain-containing polypeptide fragment consisting of a polypeptide comprising a glycosylated amino acid,
   - a second polypeptide fragment present on the N-terminal side of the sugar chain-containing polypeptide fragment and consisting of a polypeptide comprising the N-terminal side of the desired glycosylated peptide,
   - a first polypeptide fragment present on the C-terminal side of the sugar chain-containing polypeptide fragment and consisting of a polypeptide comprising the C-terminal side of the desired glycosylated peptide,
   - when it may be present, a polypeptide fragment between the sugar chain-containing polypeptide fragment and the second polypeptide fragment, and
   - when it may be present, a polypeptide fragment between the sugar chain-containing polypeptide fragment and the first polypeptide fragment,
   wherein the N-terminal of the first polypeptide fragment is designed to be a cysteine;
(2) A step of obtaining a polypeptide having said structure by employing an expression vector comprising a nucleotide sequence encoding a polypeptide having the following structure:
   (N-terminal) second polypeptide fragment-Cys-W-(His)n-Z-Met-first polypeptide fragment (C-terminal)
   [wherein n means an integer from 0 - 10, Cys is cysteine, W means any 1, 2, or 3 amino acids, Z means any 0, 1, or 2 amino acids, His means histidine, Met means methionine, and the N-terminal of the first polypeptide fragment is cysteine]
   and allowing expression from *E. coli*;
(3) A step of reacting the polypeptide obtained in step (2) with CNBr to obtain the following polypeptide fragments:
   (A) a first polypeptide fragment having cysteine at the N-terminal and
   (B) a third polypeptide fragment having the following structure:
      (N-terminal) second polypeptide fragment-Cys-W-(His)n-Z-Met' (C-terminal) (wherein Met' means a Met derivative);
(4) A step of reacting said third polypeptide fragment with a compound represented by the following formula (I): 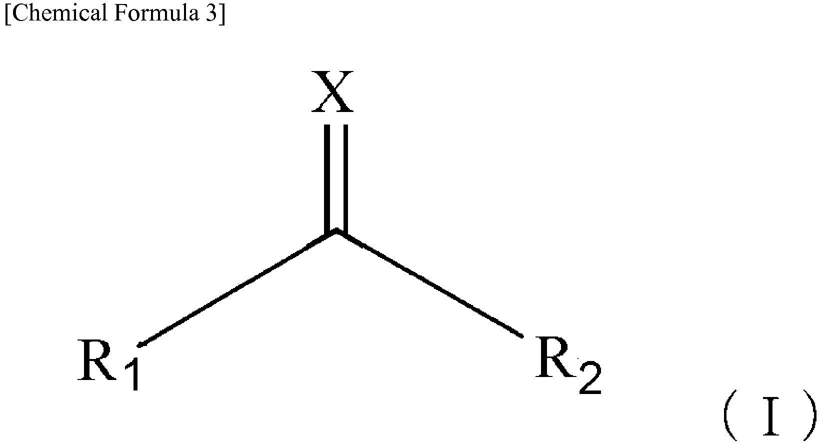 [wherein X is a sulfur atom or an oxygen atom, and R₁ and R₂ are leaving groups] and subsequently, in an organic solvent, reacting with a compound represented by the following formula (II): 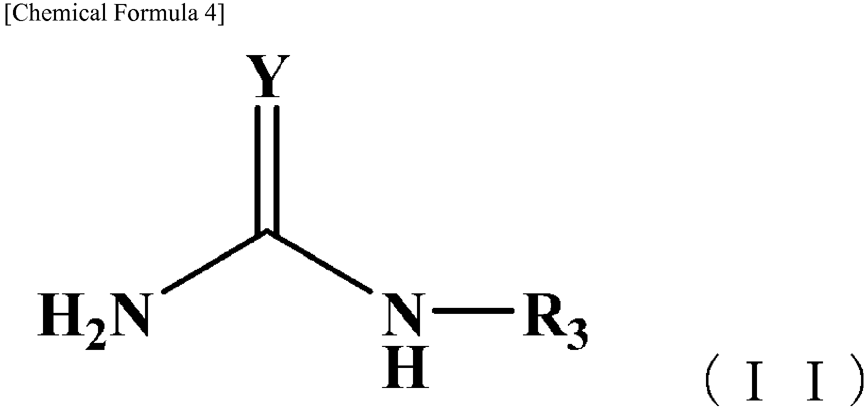 [wherein
   Y is an oxygen atom, a sulfur atom, or a NH group, and
   R₃ is a hydrogen atom, an acyl group, or an alkoxycarbonyl group] to obtain a second polypeptide fragment having the C-terminal modified having the following structure:
      (N-terminal) second polypeptide fragment-C(=O)-NH-C(=Y)NHR₃ (C-terminal);
(5) Arbitrarily, a step of reacting the second polypeptide fragment having the C-terminal modified obtained in said step (4) with further a thiol represented by the following formula

   R₄-SH

   [wherein R₄ is any one group selected from the group consisting of a substituted or unsubstituted benzyl group, a substituted or unsubstituted aryl group, and a substituted or unsubstituted alkyl group] and exchanging the -NH-C(=Y)NHR₃ group at the C-terminal with the thiol group to obtain a second polypeptide fragment having the C-terminal modified having the following structure:
   (N-terminal) second polypeptide fragment-C(=O)-SR₄ (C-terminal); and
(6) A step of linking:
   - said sugar chain-containing polypeptide fragment,
   - when it may be present, said polypeptide fragment between the sugar chain-containing polypeptide fragment and the second polypeptide fragment, and
   - when it may be present, said polypeptide fragment between the sugar chain-containing polypeptide fragment and the first polypeptide fragment separately prepared by chemical synthesis with:
      the first polypeptide fragment having cysteine at the N-terminal obtained in step (3) and
      the second polypeptide fragment having the C-terminal modified obtained in step (4) or (5)
      in an order that will give the desired glycosylated polypeptide by ligation.

Moreover, one embodiment of the method for manufacturing the glycosylated polypeptide of the present invention is characterized in that X in the compound represented by said formula (I) is a sulfur atom.

Moreover, one embodiment of the method for manufacturing the glycosylated polypeptide of the present invention is characterized in that R₁ in the compound represented by said formula (I) is an -O-C₆ aryl group.

Moreover, one embodiment of the method for manufacturing the glycosylated polypeptide of the present invention is characterized in that R₂ in the compound represented by said formula (I) is a halogen atom or a substituted or unsubstituted -S-C₆₋₁₀ aryl group.

Moreover, one embodiment of the method for manufacturing the glycosylated polypeptide of the present invention is characterized in that Y in the compound represented by said formula (II) is =NH.

Moreover, one embodiment of the method for manufacturing the glycosylated polypeptide of the present invention is characterized in that R₃ in the compound represented by said formula (II) is an acetyl group.

Moreover, one embodiment of the method for manufacturing the glycosylated polypeptide of the present invention is characterized in that W is one amino acid, and is any one amino acid selected from the group consisting of Val, Ile, Leu, and Trp.

Moreover, one embodiment of the method for manufacturing the glycosylated polypeptide of the present invention is characterized in that n is 6 - 10.

### Effects of the Invention

A method for efficiently manufacturing a first polypeptide fragment having cysteine at the N-terminal and a second polypeptide fragment having the C-terminal modified which are suitable for the NCL method from a polypeptide chain was provided by the present invention.

Moreover, with the present invention, a peptide fragment available for ligation can be expressed when linked to another peptide fragment even if it cannot be properly expressed as a peptide fragment, and it will be possible to efficiently prepare multiple peptide fragments.

Moreover, by combining the method of the present invention with conventional peptide synthesis methods, it is possible to easily manufacture even a long chain peptide having modification in a portion of the peptide which has been heretofore difficult to synthesize by e.g. using the method of the present invention to prepare the fragment without modification by biosynthesis which allows easier synthesis of a relatively long chain, the fragment with modification with solid phase synthesis, and then linking them.

More specifically, if the modification is a sugar chain, it is possible to easily manufacture a longer sugar chain peptide by preparing only the fragment comprising an amino acid having a native sugar chain linkage added by chemical synthesis, preparing the other portions by biosynthesis, and then preparing and linking a peptide fragment suitable for ligation with the method of the present application.

Moreover, a method of post-addition of a sugar chain etc. to a peptide chain via a linker is well-known, and post-addition of a sugar chain to a biosynthesized long chain peptide is also possible. However, this sugar chain binding method via a linker binds a sugar chain etc. by utilizing a particular amino acid or structure. Accordingly, for example, when multiple sites to which a sugar chain can bind exist in a peptide, it is also possible to more easily site-specifically glycosylate compared to convention by obtaining a long chain peptide by biosynthesis, cleaving out a peptide fragment comprising only the desired binding portion from the long chain peptide with the method of the present application and adding a sugar chain, and then re-linking it with the remaining portion.

As shown above, the peptide thioesterification method of the present invention is useful for protein synthesis in general.

### Brief Description of the Drawings

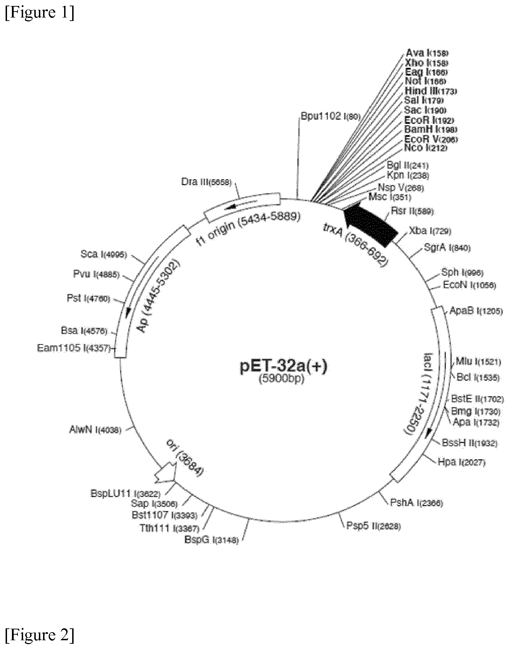

Figure 1 shows the sequence information of pET32a vector used for the expression of a polypeptide in an embodiment of the present invention.

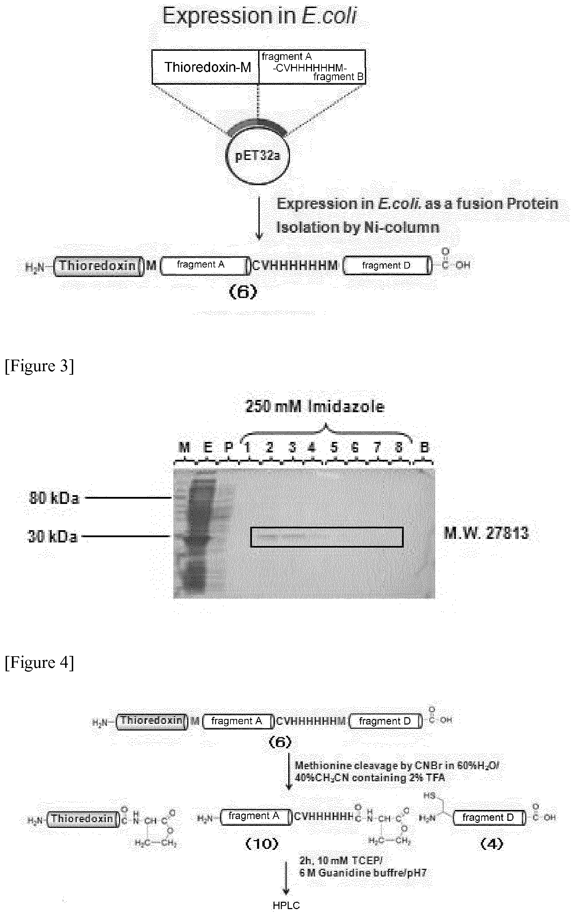

Figure 2 is a schematic diagram showing the biosynthesis of a polypeptide fragment having the intervening sequence of the present invention with *E. coli* in an embodiment of the present invention.
Figure 3 shows a photograph of the expressed protein purified with a Ni column. In Figure 3, M = molecular weight marker, E = the expressed mixture, P = the fraction that flowed through the column, 1 - 8 is the fraction number eluted with 250 mM imidazole, and B = blank.
Figure 4 is a flow chart showing a step of cleaving a polypeptide fragment having an intervening sequence expressed from *E. coli* into two polypeptide fragments with CNBr in an embodiment of the present invention. Note that thioredoxin is added via methionine to the N-terminal of the polypeptide fragment expressed from *E. coli,* and thioredoxin is also cleaved off when treated with CNBr.

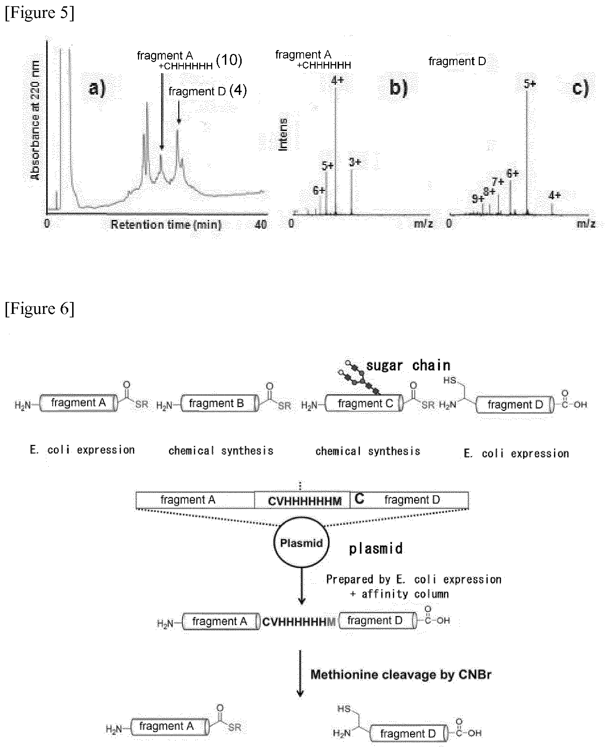

Figure 5a) shows the result of analyzing the degradation product after CNBr treatment by HPLC. Figure 5b) shows the mass spectrometry result of peptide fragment A having amino acids 1 - 27 of an interleukin-13 derivative. Figure 5c) shows the mass spectrometry result of peptide fragment D having amino acids 56 - 112 of an interleukin-13 derivative.
Figure 6 is a schematic diagram showing an embodiment utilizing the method for manufacturing the polypeptide fragment of the present invention suitable for the NCL method, showing the flow of dividing an interleukin-13 derivative into four polypeptide fragments and biosynthesizing fragments A and D as a fusion protein having an intervening sequence.

### Description of Embodiments

The preferred embodiments of the present invention will now be described.

The present invention comprises a step of reacting a polypeptide having the following structure (i)
(N-terminal) second polypeptide fragment-Cys-W-(His)n-Z-Met-first polypeptide fragment (C-terminal) ... (i)
[wherein n means an integer from 0 - 10, Cys is cysteine, W means any 1, 2, or 3 amino acids, Z means any 0, 1, or 2 amino acids, His means histidine, Met means methionine, and the N-terminal of the first polypeptide fragment is cysteine]
with CNBr to obtain the following polypeptide fragments:
(A) a first polypeptide fragment having cysteine at the N-terminal and
(B) a third polypeptide fragment having the following structure (ii):
   (N-terminal) second polypeptide fragment-Cys-W-(His)n-Z-Met' (C-terminal) ... (ii) [wherein Met' means a Met derivative].

A "peptide" herein is not particularly limited as long as it is two or more amino acids bound by an amide bond, and includes well-known peptides and novel peptides as well as altered peptides. Those generally referred to as a protein is also included in a peptide herein. Moreover, a "polypeptide" is similarly included in a peptide herein. The peptide chain employed in the method of the present invention may be a natural protein, or it may be a peptide chain obtained by a method such as biosynthesis, chemical synthesis, or cell-free synthesis.

An "altered peptide" herein includes a natural variant, a post-translationally modified form, or an artificially altered compound of the peptide. Examples of such alterations include, e.g., alkylation, acylation (such as acetylation), amidation (such as amidation of the peptide C-terminal), carboxylation, ester formation, disulfide bond formation, glycosylation, lipidation, phosphorylation, hydroxylation, and binding of a labeling component of/to one or more amino acid residues of the peptide.

An "amino acid" herein is employed in its broadest meaning, and includes not only natural amino acids, e.g. serine (Ser), asparagine (Asn), valine (Val), leucine (Leu), isoleucine (Ile), alanine (Ala), tyrosine (Tyr), glycine (Gly), lysine (Lys), arginine (Arg), histidine (His), aspartic acid (Asp), glutamic acid (Glu), glutamine (Gin), threonine (Thr), cysteine (Cys), methionine (Met), phenylalanine (Phe), tryptophan (Trp), and proline (Pro), but also non-natural amino acids such as amino acid variants and derivatives. Those skilled in the art will recognize in light of this broad definition that examples of amino acids herein include, e.g., L-amino acids; D-amino acids; chemically modified amino acids such as amino acid variants and derivatives; amino acids that are not constituents of a protein *in vivo* such as norleucine, β-alanine, and ornithine; and chemically synthesized compounds having amino acid properties well-known to those skilled in the art.

A "polypeptide fragment" herein is a polypeptide comprising a portion of the amino acid sequence of the protein of interest that is manufactured when synthesizing the protein of interest.

Here, a "first polypeptide fragment" and a "second polypeptide fragment" are fragments each comprising a portion of the amino acid sequence of the protein of interest. The "first polypeptide fragment" and the "second polypeptide fragment" herein are synthesized in a state linked via an intervening sequence. Moreover, in the method of the present invention, the "first polypeptide fragment" and the "second polypeptide fragment" can each be obtained ultimately as a polypeptide fragment in a form suitable for ligation.

The first polypeptide is a polypeptide that is designed to comprise cysteine at its N-terminal. This, when ultimately cleaved out as the first polypeptide fragment, will enable it to be ligated to another peptide fragment at the N-terminal. Moreover, the second polypeptide fragment, when ultimately cleaved out as the second polypeptide, is modified at the C-terminal in a form suitable for ligation, and this enables it to be ligated to another polypeptide fragment at the C-terminal.

Moreover, polypeptide (i) of the present invention has an intervening sequence "(N-terminal)-Cys-W-(His)n-Z-Met-(C-terminal)" between the first and second polypeptide fragments.

Here, n means an integer from 0 - 10, Cys is cysteine, W means any 1, 2, or 3 amino acids, Z means any 0, 1, or 2 amino acids, His means histidine, Met means methionine, and the N-terminal of the first polypeptide fragment is cysteine.

Moreover, polypeptide (i) may be linked to yet another polypeptide fragment at its N- and/or the C-terminal via the above intervening sequence.

In an intervening sequence, an amino acid shown by W preferably includes any one amino acid selected from the group consisting of Val, Ile, Leu, and Trp, and in particular, it is preferred that the amino acid directly adjacent to the Cys at the N-terminal is selected from these amino acids. In this way, by placing a bulky amino acid adjacent to Cys, the occurrence of a side reaction in reaction (b) employing a compound represented by formula (II) described below can be suppressed, and the yield of the second polypeptide having the C-terminal modified of interest can be improved.

Moreover, in an intervening sequence, (His)n shows a His tag, which facilitates the purification of the biosynthesized polypeptide. The purification of a polypeptide having a His tag added can be performed by a method well-known to those skilled in the art. For example, it can be easily purified by employing a commercially available Ni-NTA agarose gel.

In the above intervening sequence, when His tag is not included (when n = 0), it is also possible express polypeptide (i) with a His tag added on the N- or C-terminal. When the intervening sequence has a His tag, n is preferably an integer from 6 - 10.

Moreover, in an intervening sequence, Met is an amino acid used for cleaving off the first polypeptide present on the C-terminal side of Met.

When cleaving out the biosynthesized polypeptide (i) at the position of Met, this can be treated with e.g. CnBr and 70% HCOOH aqueous solution at room temperature. When Ser or Thr having an OH group in the side chain are contained in a large quantity in the peptide sequence to be treated, formation of the formic acid ester of the side chain will become a problem. In such a case, in order to avoid esterification, it is preferred to take measures such as reducing the formic acid content of the reaction solution, using an inorganic acid, or using TFA in order to form easily removable trifluoroacetic acid ester.

Examples of a preferred acid used in the above reaction include, but are not limited to, e.g. formic acid, phosphoric acid, trifluoroacetic acid (TFA), tribromoacetic acid, and methanesulfonic acid. These acids are preferably added at a concentration of 0.1% - 50%, more preferably 1% - 20%, and further preferably 1% - 5%.

In addition, a water-miscible solvent can also be used in the above reaction. The water-miscible solvent is not particularly limited as long as it is a solvent having water-miscibility, and can include, e.g., acetonitrile, trifluoroethanol, dimethylformamide, dimethyl sulfoxide (DMSO), and methylene chloride, and among these, acetonitrile, trifluoroethanol, and dimethylformamide are preferred. Such a solvent is preferably added at a concentration of 1% - 70%, more preferably 20% - 60%, and further preferably 35% - 50%.

In this way, the production rate of the polypeptide of interest can be increased by cleaving in the presence of an acid and a water-miscible solvent.

When polypeptide (i) was cleaved off at the position of Met by the above reaction, a polypeptide fragment having the amino acid sequence of the second polypeptide fragment -Cys-W-(His)n-Z-Met' at the N-terminal and the first polypeptide fragment at the C-terminal can be obtained.

Met' means a Met derivative, and shows a Met derivative produced by the cleaving reaction as above.

In regards to the "first polypeptide fragment" and the "second polypeptide fragment" comprised in polypeptide (i) of the present invention, a preferred fragment can be designed as appropriate based on the length of the amino acid sequence or the type of amino acids. When designing a polypeptide fragment in the present invention, the designing is done particularly so that the polypeptide fragment biosynthesized as the first polypeptide fragment has cysteine at its N-terminal.

Polypeptide (i) of the present invention may be a natural protein, or may be a peptide chain obtained by a method such as biosynthesis, chemical synthesis, or cell-free synthesis, but is preferably a recombinant protein expressed in a bacteria or a cell. The recombinant protein may be those having the same peptide sequence as a natural protein, or may be a peptide sequence having a modification such as a mutation or tag for purification, as long as it is expressed artificially expressed in a bacteria or a cell.

Polypeptide (i) of the present invention can be prepared by a method well-known to those skilled in the art. For example, it can be expressed by introducing the gene of interest into a recombinant vector and allowing expression. The recombinant vector employed herein may be any that may transform a host cell, and animal viral vectors such as a plasmid for *E. coli,* a plasmid for *Bacillus subtilis,* a plasmid for yeasts, retrovirus, vaccinia virus, and baculovirus etc. are employed depending on the host cell. Those having a regulatory sequence such as a promoter that may appropriately express the protein in the host cell are preferred. Moreover, the host cell may be any that can express a foreign gene in a recombinant vector, and *E. coli, Bacillus subtilis,* yeast, an insect cell, and an animal cell etc. are generally employed.

The method employed for transfecting the host cell with a recombinant vector may be those routinely used in general, and e.g. heat shock method, calcium chloride method, or electroporation method for *E. coli,* and lithium chloride method or electroporation for yeasts can be utilized. Moreover, transformation of an animal cell can be performed with a physical method such as electroporation, or a chemical method such as liposome method or calcium phosphate method, or a viral vector such as a retrovirus. Moreover, after the introduction of the vector, it is preferred to confirm that the DNA sequence of interest is properly integrated by a method well-known to those skilled in the art. In regards to the culturing format of the transformant host cell, the culture condition may be selected in light of the nutrient physiological nature of the host.

The peptide used in the present invention is preferably purified. The purification method of the peptide can be performed by an ordinary general purification. For example, in the case of a recombinant protein, after culturing the bacteria or cell expressing the recombinant protein used herein, a crude extract of the peptide is prepared by collecting the bacteria or cell with a well-known method, suspending this in an appropriate buffer, destructing the bacteria or cell by e.g. ultrasonic wave, lysozyme and/or freeze-thawing, and then subjecting to centrifugal separation or filtration. The buffer may include a protein denaturing agent such as urea or guanidine hydrochloride, or a surfactant such as Triton X-100™. The purification of the extract obtained in this way or the peptide comprised in the culture supernatant can be performed by a well-known purification method. For example, affinity chromatography, ion exchange chromatography, filter, ultrafiltration, gel filtration, electrophoresis, salt precipitation, and dialysis etc. are appropriately selected and combined to enable separation and purification of the peptide.

Moreover, in order to facilitate the purification of the recombinant protein, various tags may also be integrated into the expression vector. Examples of a tag that can be used include tags known to those skilled in the art such as a tag that improves expression efficiency or a tag that improves purification efficiency, and examples include, e.g. thioredoxin, a GST tag, a Myc tag, a FLAG tag, and a maltose-binding protein (MBP).

Moreover, these tags can also be linked to polypeptide (i) via Met (methionine). By preparing a tag linked to polypeptide (i) via methionine as described, the tag can be simultaneously cleaved out when cleaving the first and second polypeptide fragments with methionine as the target.

Moreover, any chemical synthesis method known in the present technical field can be employed for synthesizing other polypeptide fragments necessary for protein synthesis that are not prepared as polypeptide (i). In particular, the synthesis of the polypeptide chain at the sugar chain binding portion is preferably a chemical synthesis in order to manufacture a peptide fragment having uniform sugar chain structure. Such a method is not limited, and examples include, e.g., liquid phase synthesis method, solid phase synthesis method, Boc method, and Fmoc method. More specifically, a method for manufacturing a glycosylated peptide fragment by using glycosylated Asn as the glycosylated amino acid and applying a well-known peptide synthesis method such as solid and liquid phase synthesis as with ordinary peptide fragment synthesis can be employed. Such a method is described in International Publication No. 2004/005330 (US 2005222382 (A1)).

In the amino acid sequence of the protein of interest, when manufacturing a peptide fragment that requires ligation at the N- and C-terminals, a thiazolidine-type cysteine or a cysteine protected by e.g. an Acm group is linked to the N-terminal in order to avoid a ligation byproduct. In other words, in the ligation step, the linking proceeds from the peptide fragment on the C-terminal of the protein of interest, and the N-terminal cysteine of the peptide fragment after linking is converted back into a ligatable state for linking the peptide fragments to the protein N-terminal in order. For example, when introducing a thiazolidine-type cysteine, it can be introduced during solid phase synthesis, or a cysteine can also be selectively thiazolidinated after synthesizing the polypeptide fragment with a method well-known to those skilled in the art.

Moreover, a sugar chain can also be added to the synthesized polypeptide chain via a functional group. Any method known in the art can be employed for such linkage of a sugar chain. Such a method is not limited, examples of which include, e.g., a method of condensing a sugar chain derivative having a group selected from the group consisting of -NH-(CO)-CH₂X, -NH-(CO)-(CH₂)_{b}-CH₂X, an isothiocyanate group, - NH-(CO)ₐ-(CH₂)_{b}-CO₂H, and -NH-(CO)ₐ-(CH₂)_{b}-CHO (wherein X is a halogen atom, a is 0 or 1, and b is an integer from 1 - 4) at the reducing terminal with the sulfhydryl group of cysteine (see WO 2005/010053), or a method of employing a coupling reagent described in WO 2005/095331.

Moreover, in order to employ the peptide fragment manufactured as a fragment of a portion of the peptide of interest in the above method in a linking step, a step of thioesterifying the C-terminal of the polypeptide to be positioned at the N-terminal is necessary before the linking step. Such thioesterification can be carried out with a method well-known to those skilled in the art, for example, it can be achieved by activating the C-terminal carboxylic acid with PyBOP and DIPEA, and then adding excess alkylthiol. When employing this method, the addition of the alkylthiol is preferably performed at a low temperature, more preferably at a temperature of 10°C - - 80°C, and more preferably at 0°C - -40°C in order to control the configuration of the α carbon of the amino acid at the fragment terminal. Moreover, the above thioesterification can also be performed by e.g. the Fmoc or Boc methods described in Yamamoto et al., J. Am. Chem. Soc. 2008, 130 (2), 501-510.

A "peptide thioester form" (hereinafter may be described simply as a thioester form) herein refers to a peptide having a carboxythioester moiety (-C=O-SR) at the C-terminal. The peptide thioester form employed herein is not particularly limited as long as it is a thioester form that can cause an exchange reaction with other thiol groups. Examples of an R group include groups exemplified by R₄ below.

Polypeptide (i) of the present invention synthesized as described can yield the following polypeptide fragments by being cleaved at the position of Met in the intervening sequence:
(A) a first polypeptide fragment having cysteine at the N-terminal and
(B) a third polypeptide fragment having the following structure (ii)
   (N-terminal) second polypeptide fragment-Cys-W-(His)n-Z-Met' (C-terminal) ... (ii) [wherein Met' means a Met derivative].

When yet another polypeptide fragment is linked to the N- and/or C-terminal of polypeptide (i) via the above intervening sequence, for example, the polypeptide fragment below can further be obtained.
(B') (N-terminal) polypeptide fragment -Cys-W-(His)n-Z-Met' (C-terminal)

The first polypeptide fragment having cysteine at the N-terminal (A) obtained as above can be ligated with a peptide having a thioester at the C-terminal. Accordingly, the present invention also provides a method for manufacturing a polypeptide comprising a step of linking a first polypeptide fragment having cysteine at the N-terminal obtained by the method of the present invention with a peptide having a thioester at the C-terminal by ligation.

Here, the method of the present invention comprises:
a step of reacting a third polypeptide fragment (ii) obtained by cleaving out polypeptide (i) with a compound represented by the following formula (I): 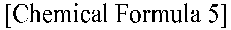 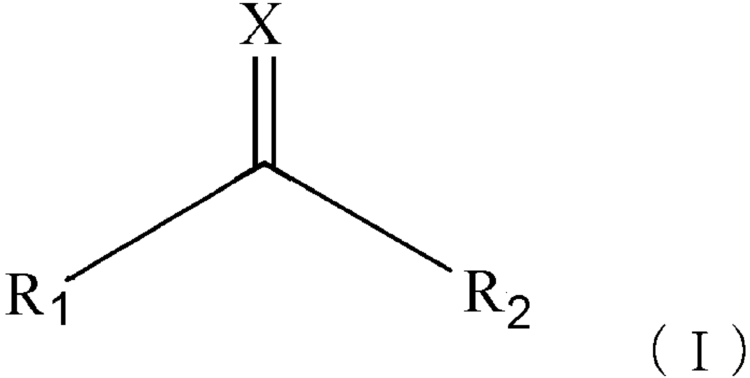 [wherein X is a sulfur atom or an oxygen atom, and R₁ and R₂ are leaving groups]
and subsequently, in an organic solvent, reacting with a compound represented by the following formula (II): 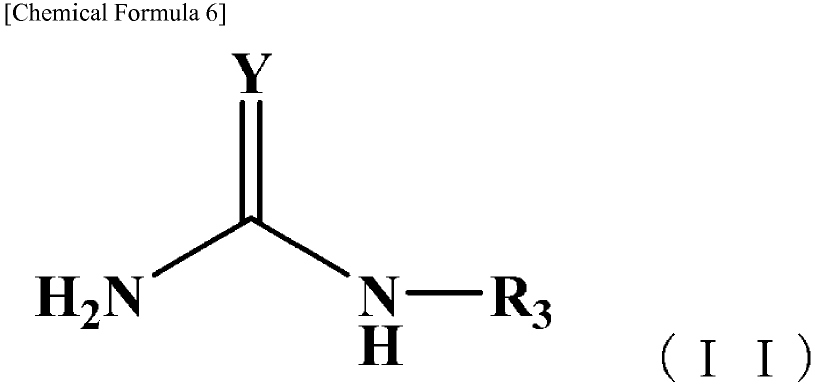 [wherein Y is an oxygen atom, a sulfur atom, or =NH, and R₃ is a hydrogen atom, an acyl group, or an alkoxycarbonyl group]
   to obtain a second polypeptide fragment having the C-terminal modified having the following structure:
   (N-terminal) second polypeptide fragment-C(=O)-NH-C(=Y)NHR₃ (C-terminal).

In the above step, a step of preparing a first intermediate by reacting the thiol group of the cysteine residue in the third polypeptide fragment (ii) having a cysteine residue with a compound represented by formula (I) (reaction a) is first performed.

The compound used in reaction (a) is shown by the following formula (I).

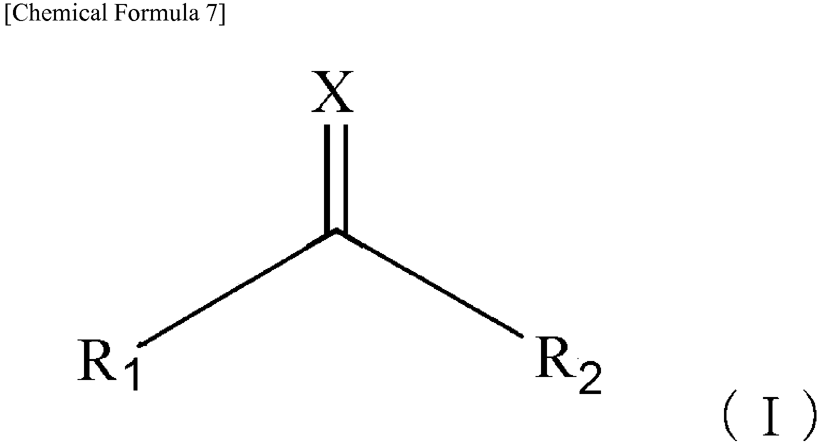

In the formula, X is a sulfur or an oxygen atom, but is preferably a sulfur atom.

R₁ and R₂ is not particularly limited as long as it has a lower nucleophilicity than the substituted atom or atomic group and has a detachable function as a leaving group under the condition of reaction (a), but it is preferred that R₁ and R₂ are each different leaving groups. Specifically, a halogen atom, a substituted or unsubstituted - O-alkyl group, a substituted or unsubstituted -O-alkenyl group, a substituted or unsubstituted -O-alkynyl group, a substituted or unsubstituted -O-aryl group, a substituted or unsubstituted -O-heteroaryl group, a substituted or unsubstituted -S-alkyl group, a substituted or unsubstituted -S-alkenyl group, a substituted or unsubstituted -S-alkynyl group, a substituted or unsubstituted -S-aryl group, or a substituted or unsubstituted -S-heteroaryl group are included as R₁ and R₂. More preferably, as R₁ and R₂, a combination of a leaving group selected from the group consisting of a substituted or unsubstituted -O-C₆₋₁₀ aryl group and a substituted or unsubstituted -S-C₁₋₈ alkyl group as R₁, and a leaving group selected from the group consisting of a halogen atom, a substituted or unsubstituted -S-C₁₋₈ alkyl group, and a substituted or unsubstituted -S-C₆₋₁₀ aryl group as R₂ is included.

An "alkyl group" herein is a monovalent group induced by removing any one hydrogen atom from an aliphatic hydrocarbon, and has a subset of hydrogen and carbon atom-containing hydrocarbyl or hydrocarbon. An alkyl group comprises a linear or branched structure. The alkyl group of the present invention preferably includes an alkyl group having 1 to 8 carbon atoms. A "C₁₋₈ alkyl group" shows an alkyl group having 1 to 8 carbon atoms, specific examples of which include, e.g., a methyl group, an ethyl group, a propyl group, a butyl group, a pentyl group, a hexyl group, a heptyl group, and an octyl group.

An "alkenyl group" herein is a monovalent group having at least one double bond. The geometrical form of the double bond can take an Entgegen (E) or Zusammen (Z) and cis- or trans- configurations depending on the configuration of double bonds and substituents. An alkenyl group comprises a linear or branched one. The alkenyl group of the present invention preferably includes an alkenyl group having 2 to 8 carbon atoms. A "C₂₋₈ alkenyl group" shows an alkenyl group having 2 to 8 carbon atoms, specific examples of which include, e.g., a vinyl group, an allyl group, a propenyl group, a butenyl group, a pentenyl group, a hexenyl group, a heptenyl group, and an octenyl group.

An "alkynyl group" herein is a monovalent group having at least one triple bond. An alkynyl group comprises a linear or branched alkynyl group. The alkynyl group of the present invention preferably includes an alkynyl group having 2 to 8 carbon atoms. A "C₂₋₈ alkynyl group" shows an alkynyl group having 2 to 8 carbon atoms, specific examples of which include, e.g., an ethynyl group, a 1-propynyl group, a 2-propynyl group, a butynyl group, a pentynyl group, a hexynyl group, a heptynyl group, and an octynyl group.

An "aryl group" herein means an aromatic hydrocarbon cyclic group. The aryl group of the present invention preferably includes an aryl group having 6 to 10 carbon atoms. A "C₆₋₁₀ aryl group" shows an aryl group having 6 to 10 carbon atoms, specific examples of which include, e.g., a phenyl group, a 1-naphtyl group, and a 2-naphtyl group.

A "heteroaryl group" herein means a monovalent or bivalent group induced by removing one or two hydrogen atoms at any position from a heteroaryl ring. A "heteroaryl ring" herein means an aromatic ring containing one or more heteroatoms in the atoms constituting the ring, and is preferably a 5 to 9-membered ring. The ring may be a monocyclic ring, or may be a bicyclic heteroaryl group condensed with a benzene ring or a monocyclic heteroaryl ring. Specific examples include, e.g., a furanyl group, a thiophenyl group, a pyrrolyl group, a benzofuranyl group, a benzothiophenyl group, an indolyl group, a pyridyl group, and a quinolinyl group.

The type and the number of the substituent and the substitution position of the leaving group described above are not particularly limited, and examples of a substituent include, e.g., an alkyl group, an alkenyl group, an alkoxy group, an aryl group, a formyl group, a carbonyl group, a carboxyl group, an alkylcarboxyl group, an alkoxycarbonyl group, a halogen, a sulfonyl group, or a nitro group.

More specifically, a compound represented by formula (I) of the present invention can include e.g. the following.

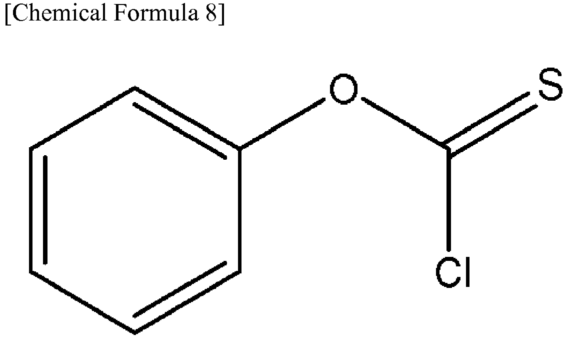

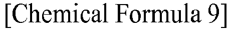

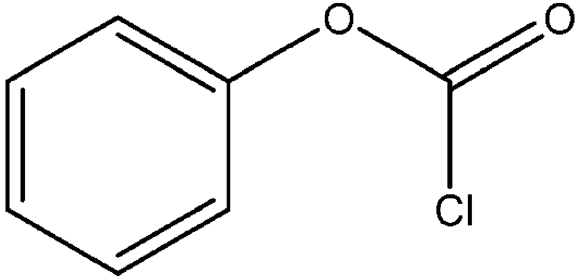

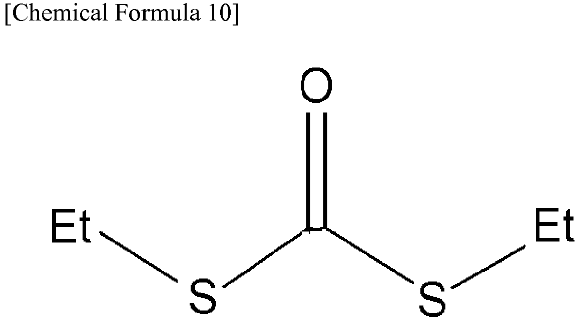

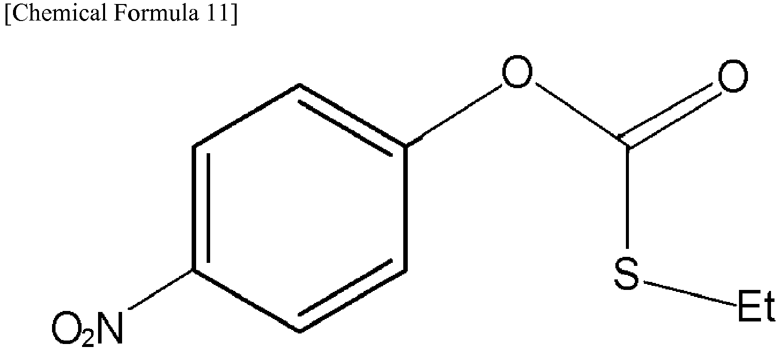

Moreover, it is also possible to employ: 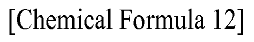 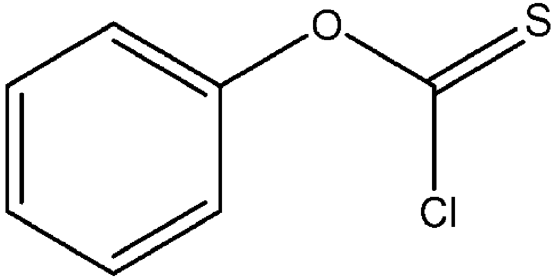 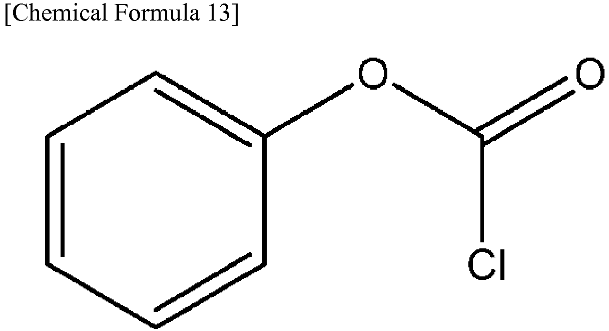 shown above reacted with MPAA ((4-carboxymethyl)thiophenol), i.e. the thionoformating reagents below.

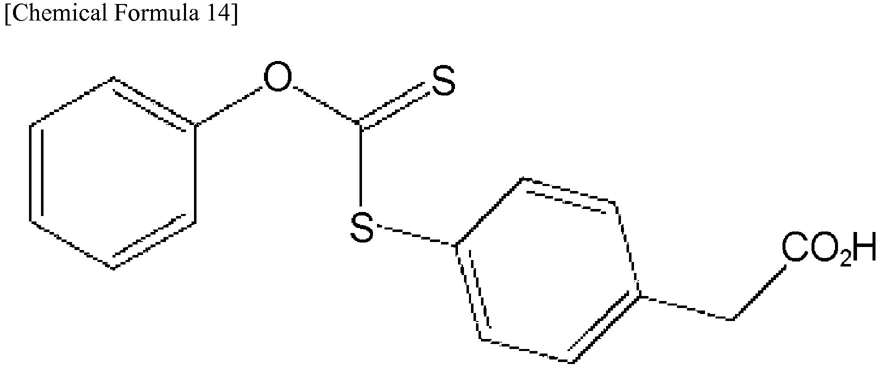

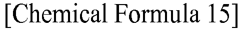

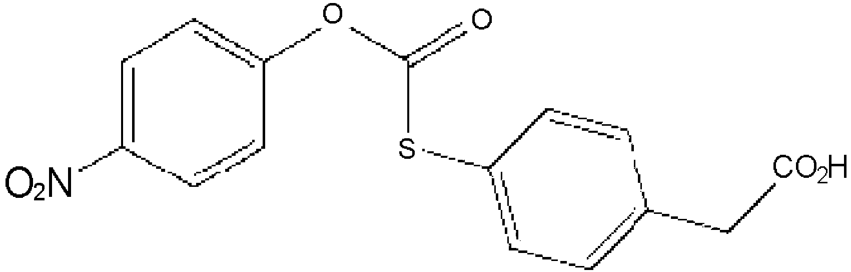

By reacting a compound represented by formula (I) with the cysteine residue of a third polypeptide fragment (ii), a first intermediate wherein a -C(=X)-R₁ group is bound to the SH group in the cysteine residue can be obtained, as in the figure below.

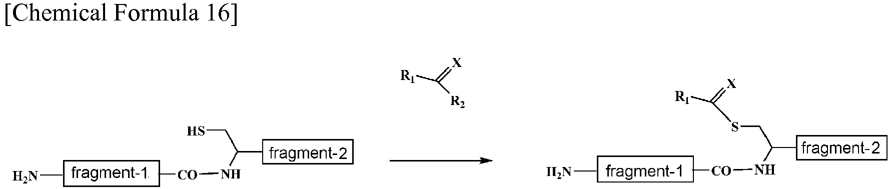

In the present invention, reaction (a) is preferably performed under an acidic condition, particularly preferably at pH 3 - 5. The reaction is preferably performed in a mixed solvent of a buffer and acetonitrile at 0 - 50°C, preferably at 15 - 25°C for about 0.1 - 3 hours, preferably for 10 minutes - 1 hour, but is not limited thereto.

Subsequently, in the method of the present invention, said first intermediate is reacted with a compound represented by formula (II) in an organic solvent, a -NH-C(=Y)NHR₃ group is added to the carboxyl group forming a peptide bond with the amino acid adjacent to the N-terminal of said cysteine residue, and said peptide bond is cleaved in order to perform a step of obtaining the peptide fragment on the N-terminal side of said cleaved peptide bond as a second intermediate (reaction (b)).

The compound used in the above reaction (b) is shown by the following formula (II).

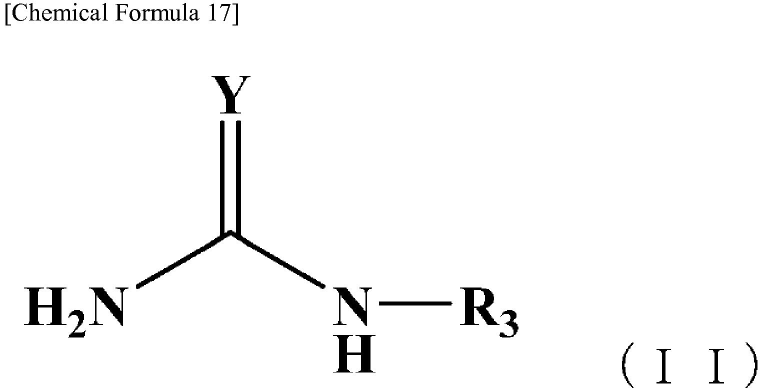

In the formula, Y is an oxygen atom, an NH group, or a sulfur atom, and R₃ is a hydrogen atom, an acyl group, or an alkoxycarbonyl group.

An "acyl group" herein means an atomic group having an OH group removed from the carboxyl group of the carboxylic acid. The acyl group of the present invention preferably includes an acyl group having 1 - 5 carbon atoms. Specific examples include, e.g., an acetyl group, a pivaloyl group, a propionyl group, and a butyloyl group.

An "alkoxy group" herein means an oxy group with an "alkyl group" bound thereto. The alkoxy group of the present invention may be linear or branched. The alkoxy group of the present invention preferably includes a linear alkoxy group having 1 to 14 carbon atoms or a branched alkoxy group having 3 to 14 carbon atoms. Specific examples can include, e.g., a methoxy group, an ethoxy group, an n-propyloxy group, an isopropoxy group, an n-butoxy group, a 2-methyl-2-propyloxy group, an n-pentyloxy group, and an n-hexyloxy group.

Moreover, a "C₂₋ₙ alkoxycarbonyl group" means a carbonyl group having C₁₋₍ₙ₋₁₎ alkoxy groups. The alkoxycarbonyl group of the present invention can preferably include an alkoxycarbonyl group having 2 to 15 carbon atoms. Specific examples can include, e.g., a methoxycarbonyl group, an ethoxycarbonyl group, an n-propyloxycarbonyl group, an isopropoxycarbonyl group, an n-butoxycarbonyl group, a 2-methyl-2-propyloxycarbonyl group, an n-pentyloxycarbonyl group, and an n-hexyloxycarbonyl group.

An acyl group preferably includes an acetyl group. Moreover, an alkoxycarbonyl group preferably includes a tert-butoxycarbonyl group (Boc group).

More specifically, examples of a compound represented by formula (II) of the present invention can include the following.

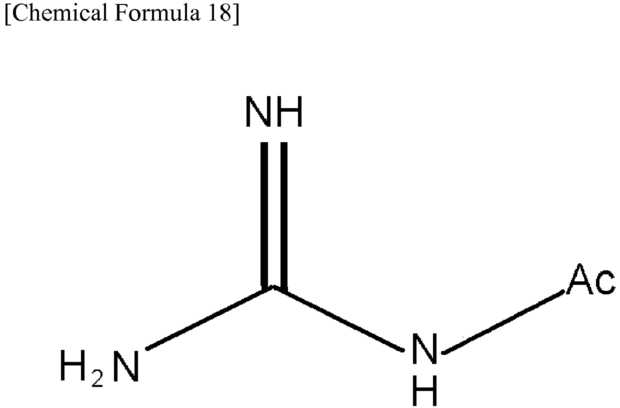

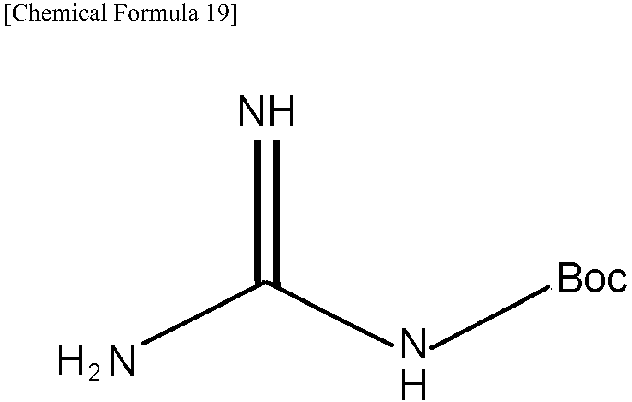

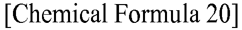

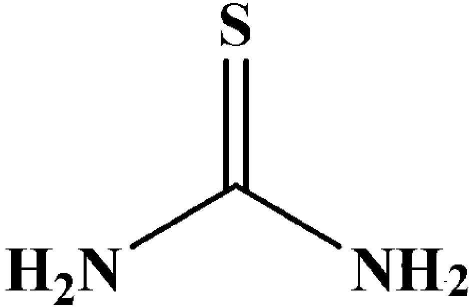

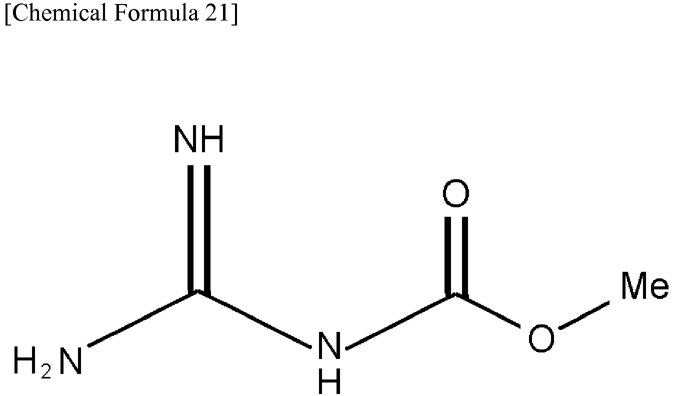

In the present invention, reaction (b) is preferably performed in the presence of an organic solvent. The preferred organic solvent is one having high solubility and low nucleophilicity. Examples of such organic solvents can include, e.g., DMSO, DMF, and dioxane. The reaction is performed at 0 - 50°C, preferably at 15 - 25°C for about 1 - 24 hours, preferably for 5 - 10 hours, but is not limited thereto.

By adding a -NH-C(=Y)NHR₃ group to the carboxyl group forming a peptide bond with the amino acid adjacent to the N-terminal of the cysteine residue, the peptide chain is cleaved at the N-terminal side of the cysteine residue as in the figure below.

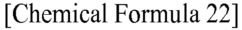

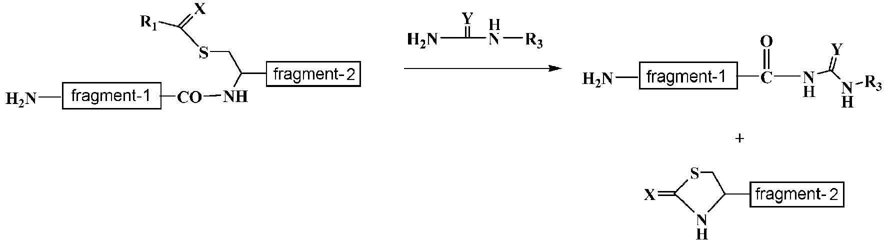

When the peptide side chain has an amino group, a lipophilic protecting group may be introduced into the side chain amino group before performing reaction (b) of the present invention. Examples of a lipophilic protecting group can include, but are not particularly limited to, a protecting group such as a carbonyl-containing group such as a 9-fluorenylmethoxycarbonyl (Fmoc) group, a t-butyloxycarbonyl (Boc) group, and an allyloxycarbonyl (Alloc) group, an acyl group such as an acetyl (Ac) group, an allyl group, and a benzyl group.

When introducing a lipophilic protecting group, e.g. when introducing an Fmoc group, introduction can be carried out by adding 9-fluorenylmethyl-N-succinimidyl carbonate and sodium hydrogen carbonate and allowing reaction. The reaction may be performed at 0 - 50°C, preferably at room temperature for approximately about 1 - 5 hours, but is not limited thereto.

In reaction (b), the peptide fragment on the N-terminal side of the cleaving site of the cleaved peptide chain can be obtained as the second intermediate having the following formula (III).

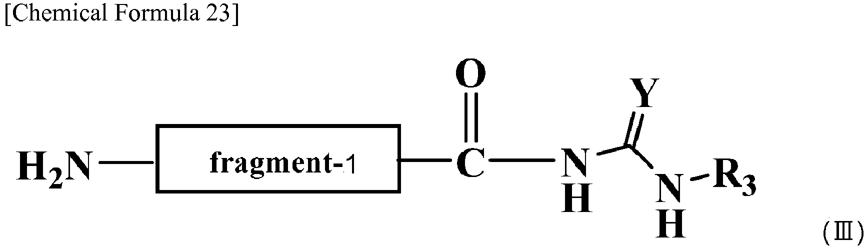

A method for manufacturing the peptide thioester form of the present invention further comprises a step of thioesterifying the C-terminal of the second intermediate by reacting said second intermediate with a thiol in order to exchange the C-terminal -NH-C(=Y)NHR₃ group with the thiol group (reaction (c)).

The second intermediate employed for reaction (c) may or may not be isolated after reaction (b).

In a preferred aspect, a thiol represented by the following formula (IV) is employed for said reaction (c).

R₄-SH (Formula IV)

R₄ is not particularly limited as long as it is a group that does not inhibit the thiol exchange reaction and becomes a leaving group in the substitution reaction on the carbonyl carbon. Preferably, R₄ is any one group selected from a substituted or unsubstituted benzyl group, a substituted or unsubstituted aryl group, and a substituted or unsubstituted alkyl group, more preferably any one group selected from a substituted or unsubstituted benzyl group, a substituted or unsubstituted C₆₋₁₀ aryl group, and a substituted or unsubstituted C₁₋₈ alkyl group. More specifically, it can be selected from a benzyl-type leaving group such as benzyl mercaptan, an aryl-type leaving group such as thiophenol and 4-(carboxymethyl)-thiophenol, and an alkyl-type leaving group such as a 2-mercaptoethanesulfonate group, 3-mercaptopropionic acid amide. The type and the number of the substituent and the substitution position of these leaving groups are not particularly limited.

By performing reaction (c), the second intermediate is completely converted into a thioester form as in the figure below.

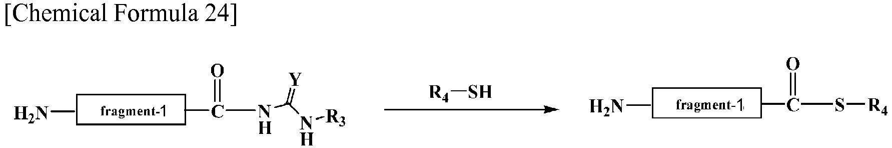

The peptide thioester form obtained as described above can be linked with a peptide comprising an amino acid residue having an -SH group at the N-terminal (or a modified peptide) among peptides or modified peptides by employing a ligation method. Accordingly, the present invention also provides a method for manufacturing a polypeptide comprising a step of linking a peptide thioester form obtained by the method of the present invention with a peptide chain having cysteine at the N-terminal by ligation.

Moreover, it is also possible to use the second intermediate obtained in said step (b) directly in the ligation method instead of the above peptide thioester form. This case is preferred in that the conversion step into the thioester form can be omitted.

The "ligation method" herein includes not only the native chemical ligation method (NCL method) described in Patent Literature 1, but also the case of applying the above native chemical ligation method to a peptide comprising a non-natural amino acid and an amino acid derivative (such as threonine derivative A, a protected methionine, and a glycosylated amino acid). A peptide having a natural amide bond (peptide bond) at the linking site can be manufactured by a ligation method.

The linking employing a ligation method can be performed between any of peptide-peptide, peptide-modified peptide, and modified peptide-modified peptide.

The terms used herein are to be employed to describe particular embodiments, and do not intend to limit the invention.

Moreover, the term "comprising" as used herein, unless the content clearly indicates to be understood otherwise, intends the presence of the described items (such as components, steps, elements, and numbers), and does not exclude the presence of other items (such as components, steps, elements, and numbers).

Unless otherwise defined, all terms used herein (including technical and scientific terms) have the same meanings as those broadly recognized by those skilled in the art of the technology to which the present invention belongs. The terms used herein, unless explicitly defined otherwise, are to be construed as having consistent meanings with the meanings herein and related technical fields, and shall not be construed as having idealized or excessively formal meanings.

The embodiments of the present invention may be described referring to schematic diagrams. In case of schematic diagrams, they may be exaggerated in presentation in order to allow clear description.

Terms such as first and second are employed to express various elements, and it should be recognized that these elements are not to be limited by these terms. These terms are employed solely for the purpose of discriminating one element from another, and it is for example possible to describe a first element as a second element, and similarly, to describe a second element as a first element without departing from the scope of the present invention.

The present invention will now be described in further detail referring to Examples. However, the present invention can be embodied by various aspects, and shall not be construed as being limited to the Examples described herein.

### Examples

The method for synthesizing an interleukin-13 (IL13) derivative (SEQ ID NO. 1) (called a "derivative" because the sugar chain differs from a natural IL13) using the method of the present invention is shown in the Examples below.

Specifically, an interleukin 13 derivative which is the desired glycosylated polypeptide to be manufactured was classified and designed as below.
- polypeptide fragment A having a sequence of amino acids 1 - 27 in IL13 (this corresponds to the "second polypeptide fragment present on the N-terminal side of the sugar chain-containing polypeptide fragment and consisting of a polypeptide comprising the N-terminal side of the desired glycosylated peptide.")
- polypeptide fragment B having a sequence of amino acids 28 - 43 in IL13 (this corresponds to the "polypeptide fragment between the sugar chain-containing polypeptide fragment and the second polypeptide fragment.")
- polypeptide fragment C having a sequence of amino acids 44 - 55 in IL13 (this comprises a glycosylated amino acid. Accordingly, this fragment corresponds to the "sugar chain-containing polypeptide fragment consisting of a polypeptide comprising a glycosylated amino acid.")
- polypeptide fragment D having a sequence of amino acids 56 - 112 in IL13 (this corresponds to the "first polypeptide fragment present on the C-terminal side of the sugar chain-containing polypeptide fragment and consisting of a polypeptide comprising the C-terminal side of the desired glycosylated peptide.")

Figure 6 is a schematic diagram showing such design.

As described below, the above polypeptide fragments A and D are expressed by an *E. coli* expression method and prepared after given steps.

Moreover, the above polypeptide fragments B and C are prepared by chemical synthesis.

Then, the IL-13 derivative is manufactured by linking these four polypeptide fragments prepared by ligation.

Note for example that the description polypeptide fragment C·D means a linked form of polypeptide fragment C and polypeptide fragment D.

### 1. Preparation of polypeptide fragment A (amino acids 1 - 27) and polypeptide fragment D (a sequence of amino acids 56 - 112)

The two non-glycosylated portions (polypeptide fragment A (compound 1) (SEQ ID NO. 2) and polypeptide fragment D (compound 4) (SEQ ID NO. 3)) were prepared with an *E. coli* expression system as a fusion protein (compound 6) (SEQ ID NO. 4).

### 1-1. Introduction of nucleic acid molecule encoding non-glycosylated portion into E. coli

(1) To a test tube containing 5 ml of LB medium A (comprising 10 g of Bacto tryptone, 5 g of yeast extract, 10 g of NaCl, and 15 g of agar in 1 1 of H₂O), 100 µl of *E*. *coli* (BL21) suspension was added, and after vortex treatment, incubated overnight at 37°C.
(2) Two test tubes containing 5 ml of LB medium A with 200 µl of the culture medium from (1) added were prepared, and incubated at 37°C. Turbidity (OD600) was measured, and the test tubes were placed in ice after 50 minutes when OD600 = 0.4.
(3) The test tube contents from (2) were transferred to a conical tube, and centrifuged at 0°C, 2,000 rpm for 20 minutes.
(4) The supernatant was disposed by decantation, the precipitate was triturated, 3 ml of 0.1 M CaCl₂ was added, and left in ice for 20 minutes.
(5) The tube from (4) was centrifuged at 0°C, 2,000 rpm for 20 minutes.
(6) The supernatant was disposed by decantation, the precipitate was triturated, 0.5 ml of 0.1 M CaCl₂ was added, and after vortex treatment, transferred to a separately prepared tube.
(7) To the tube from (6), 2 ml of pET32a vector comprising a nucleic acid molecule encoding a sequence which is a sequence of amino acids 1 - 27 of IL13, an intervening sequence (Cys-Val-His-His-His-His-His-His-Met), and a sequence of amino acids 56 - 112 linked together (SEQ ID NO. 5) was added, lightly mixed, and then left in ice for 1 hour. The vector is freely distributed to third parties by the inventors thereof. Said nucleic acid molecule is inserted at the NcoI/BamHI site downstream of thioredoxin in the pET32a vector (Novagen, Inc.) (see Figure 1).
(8) The sample from (7) was floated in a water bath at 42°C for 3 minutes (heat shock method), and subsequently cooled in ice for 1 minute.
(9) To (8) was added 5 ml of LB medium A, and after incubating at 37°C for 45 minutes, this was centrifuged at room temperature, 3,000 rpm for 10 minutes.
(10) After the supernatant (filtrate) was disposed and the precipitate was triturated, 1 ml of LB medium A was added to the precipitate, and after vortex treatment, the total volume was divided into 100 µl and 900 µl. Each was spread on a petri dish plated with 30 ml of LB medium B (10 g of Bacto tryptone, 5 g of yeast extract, 5 g of NaCl, and 5 g glucose were mixed in 1 l of H₂O, 1.5% agar (15 g/1) was added, this was autoclaved for 20 minutes, allowed to cool to 50 - 60°C, and 100 mg of ampicillin was added) with a bacteria spreader.
(11) The petri dishes from (10) were left at 37°C for 10 hours.
(12) Colonies formed by (11) were picked up into a test tube (containing 3 ml of LBamp medium comprising LB medium A with 10% ampicillin at a ratio of 1000:1) with a sterilized toothpick, and after vortex treatment, incubated at 37°C overnight.

### 2. Confirmation of gene

### 2-1. Extraction of plasmid DNA

(1) 1.5 ml of the sample from the above 1-1. (12) was transferred to an Eppendorf tube.
(2) After centrifugation at room temperature, 7,000 rpm for 3 minutes, the supernatant was removed and the precipitate was triturated.
(3) To the precipitate was added 100 ml of GTE (50 mM glucose, 25 mM Tris·HCl, and 10 mM EDTA), and after vortex treatment, suspended for 5 minutes with a shaker, 200 ml of alkali SDS solution (0.2N NaOH, 1% SDS) was further added and mixed by inversion, and then cooled in ice for 5 minutes.
(4) 150 ml of 5 M potassium acetate solution was added, mixed by inversion, and then cooled in ice for 5 minutes.
(5) 15 ml of chloroform was added and subjected to vortex treatment, centrifuged at 4°C, 13,000 rpm for 15 minutes, and then the supernatant was transferred to another Eppendorf tube, to this supernatant was added 3 ml of 10 mg/ml RNase, and left at 37°C for 1 hour.
(6) To the sample from (5) was added 500 ml of phenol:chloroform:isoamylalcohol = 25:24:1, and centrifuged at room temperature, 13,000 rpm for 10 minutes.
(7) The supernatant was transferred to another Eppendorf tube, 40 ml of 3 M NaOAc and 1 ml of EtOH were added and mixed by inversion, and after vortex treatment, left at -80°C for 30 minutes.
(8) The Eppendorf tube from (7) was centrifuged at 4°C, 13,000 rpm for 30 minutes.
(9) The supernatant was removed, 1 ml of 70% EtOH was added to the precipitate, and after vortex treatment, centrifuged at room temperature, 13,000 rpm for 5 minutes.
(10) The supernatant was removed and dried in a desiccator.
(11) 15 ml of autoclaved pure water (aH₂O) was added, and stirred with a shaker for 10 minutes.

### 2-2. Restriction enzyme treatment and electrophoresis of plasmid DNA

(1) 5 ml of the sample from the above 2-1. (11) was taken, 15 ml of restriction enzyme treatment solution A comprising BamHI and NcoI (2.25 ml of 10 x K buffer (200 mM Tris·HCl pH 8.5, 100 mM MgCl₂, 10 mM DTT, and 1 M KCl), 2.25 ml of 0.1% BSA, 0.85 ml of NcoI, 0.85 ml of BamHI, and 10.76 ml of aH₂O) was added and mixed, and left at 37°C for 2 hours.
(2) This was cooled to room temperature, and 5 ml of a quenching solution (50% glycerol, 0.5% SDS, 2 mM EDTA, 0.25% xylene cyanol, and 0.25% bromophenol blue in H₂O) was added.
(3) The sample from (2) was loaded onto a 4% polyacrylamide gel (1.995 ml of 30% acrylic solution, 1.5 ml of 10 x TBE (108 g of Trisma base, 55 g of boric acid, and 80 ml of 0.25 M EDTA in 1 1 of H₂O), 75 ml of 10% APS (0.1 g of ammonium persulfate in 1 1 of H₂O), 10 ml of TEMED, and 15 ml of aH₂O), and electrophoresis was performed at 50 V (constant voltage). (1 x TBE (10 x TBE diluted 10-folds) was employed as the running buffer and Φ x 174 was employed as the molecular weight marker.)
(4) This was stained with ethidium bromide for 15 minutes, and the sample was evaluated at 300 nm to observe a band of approximately 759 bp. Since the protein to be expressed is 253 amino acids chain length, it was confirmed that a DNA having a corresponding length was cleaved out.

### 3. Expression of protein

(1) 5 ml of 2 x YTamp (16 g of Bacto tryptone, 10 g of Bacto yeast extract, 5 g of NaCl, and 100 mg of ampicillin in 1 1 of aH₂O) were placed into each of two test tubes, 30 ml of the solution from the above 1-1. (12) (a sample stored at 4°C for 2 weeks) was added to each, and then incubated at 37°C overnight.
(2) To 1 l of 2 x YTamp was added 10 ml of the solution prepared in (1), and incubation was started at 37°C.
(3) Turbidity (OD600) was measured, and after 2 hours when OD = 0.6, 1 ml of 1 M isopropyl-β-D-thiogalactopyranoside (IPTG) was added, and induction was performed for 3 hours.
(4) The sample from (3) was soaked in ice, and centrifuged at 4°C, 3,500 rpm for 10 minutes.
(5) The supernatant was disposed, and bacteria was collected.
(6) This was stored at -20°C.

### 4. Purification of expressed protein

(1) The sample obtained in the above 3. (6) was thawed at room temperature, and then dissolved in 100 ml of buffer A (100 mM NaH₂PO₄, 10 mM Tris·HCl, 6 M guanidine hydrochloride, and 5 mM imidazole, pH 8.0), and sonicated in ice to break the cell wall.
(2) 10 ml of Ni-NTA agarose (50% EtOH) was taken, filled in a solid phase synthesis tube (column), and then substituted with buffer A. The sample from (1) was loaded, mixed sufficiently with Ni-NTA agarose and then eluted.
(3) The eluate was collected, and re-loaded in the column. This operation was repeated 10 times, and the final eluate was collected. (permeate (1))
(4) The Ni-NTA agarose from (3) was washed with 100 ml of buffer B (100 mM NaH₂PO₄, 10 mM Tris·HCl, 6 M guanidine hydrochloride, and 250 mM imidazole, pH 8.0), and the wash liquid was collected. (permeate (2))
(5) Elution was performed with 10 ml of buffer C (100 mM NaH₂PO₄, 10 mM Tris·HCl, 6 M guanidine hydrochloride, and 400 mM imidazole, pH 8.0) (eluate). The elution of the protein was confirmed with CBBG solution (Coomassie Brilliant Blue G250).
(6) The eluate from (5) was placed in a dialysis tube, the dialysis tube was floated in a 5 1 beaker containing pure water, and stirred at 4°C overnight in order to perform dialysis.
(7) The dialysis tube content was collected in a centrifuge tube, centrifuged at 4°C, 3,500 rpm for 10 minutes, and then the supernatant was disposed to yield about 25 mg of polypeptide fragment A-Cys-Val-His tag-Met-polypeptide fragment D fusion polypeptide bound to a thioredoxin protein (compound 6) (see Figures 2 and 3). 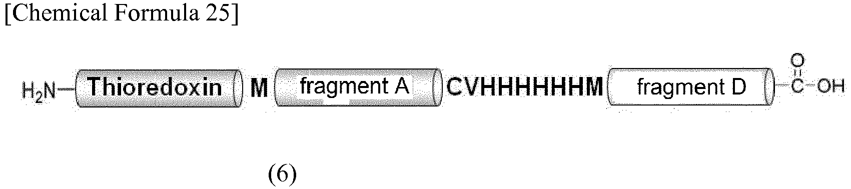 (C indicates Cys, V indicates Val, H indicates His, and M indicates Met. The same applies below.)

### 5. CNBr treatment

(1) To 100 mg of the sample of the fusion polypeptide (compound 6) obtained in 4. (7) after purification with HPLC and lyophilization was added 20 ml of an aqueous solution containing 2% TFA and 40% acetonitrile under argon, 76 mg of CNBr was further added, protected from light, and stirred overnight. By this treatment, the amide bond at the C-terminal of methionine between the fusion protein which is the thioredoxin portion + the His tag and the non-glycosylated portion which is polypeptide fragment D is hydrolyzed, and polypeptide fragment A with an intervening sequence added and polypeptide fragment D can be separated.
(2) This was analyzed with HPLC, and mass spectrometry confirmed that 2.3 mg of the desired polypeptide fragment A-C-Val-His-His-His-His-His-His-Met derivative (compound 10) (SEQ ID NO. 6) and 4.2 mg of polypeptide fragment D (compound 4) were obtained at a retention time of 12 minutes (see Figures 4 and 5).

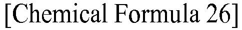

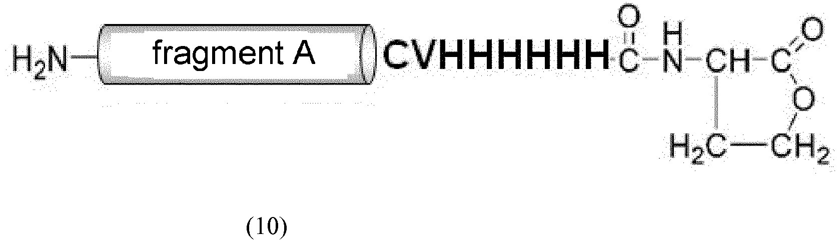

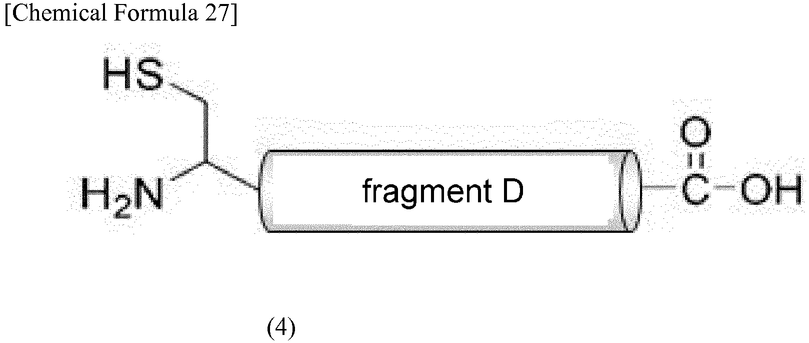

### 6. Thioesterification

After dissolving 6 mg of the polypeptide fragment A-C-Val-His-His-His-His-His-His-Met derivative (compound 10) obtained in the above 5. in 680 µL of dimethylformamide (DMF), 2.6 µL (10 eq) of N,N-diisopropylethylamine (DIEtN) and 3.2 mg (10 eq) of Boc-OSu were added to the solution and reacted for about 1 hour to yield a 37-residue partially protected polypeptide fragment having Boc group at a total of two sites at the N-terminal amino group of the polypeptide fragment A-C-Val-His-His-His-His-His-His-Met derivative (compound 10) and the side chain amino group of the Lys residue in the sequence (compound 100) (SEQ ID NO. 7). This 37-residue peptide was dissolved in phosphate buffer (pH = 5.0, 100 nM), the chlorothionoformate (5 equivalents) was added, the thiol group of cysteine was modified as a thiocarbonate-type, and directly purified with HPLC. The polypeptide fragment obtained (compound 101) (SEQ ID NO. 8) was lyophilized to yield 1.7 mg. This total amount was dissolved in DMSO, acetylguanindine (0.5 M in DMSO) was added, and reaction was allowed at ambient temperature for 8 hours. The product was purified with HPLC to obtain 0.3 mg of a polypeptide fragment having the C-terminal carboxyl group of polypeptide fragment A having a sequence of amino acids 1 - 27 of IL-13 modified with a guanidino group (compound 102) (SEQ ID NO. 9).

This peptide-guanindine derivative can be treated with an alkylthiol such as mercaptoethyl sulfuric acid to convert into a thioester derivative, but it can also be employed for native chemical ligation directly as guanidino group. 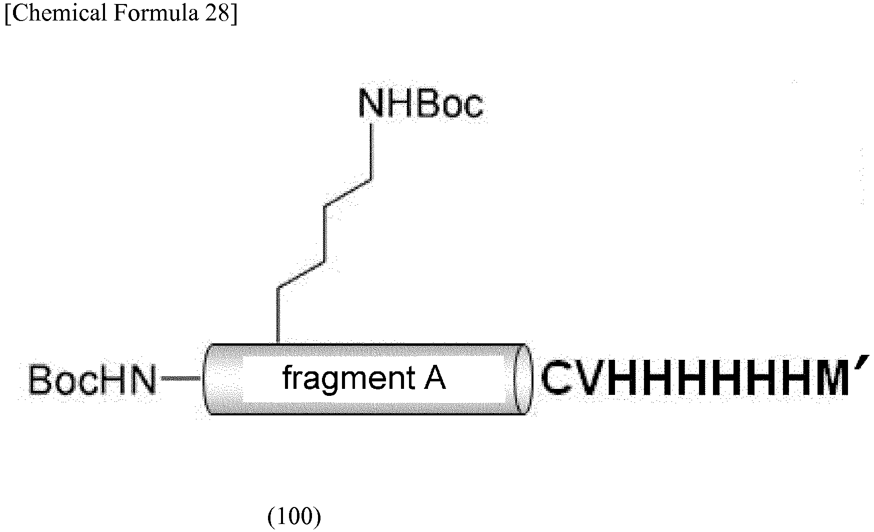 (M' indicates a Met derivative. The same applies below.)

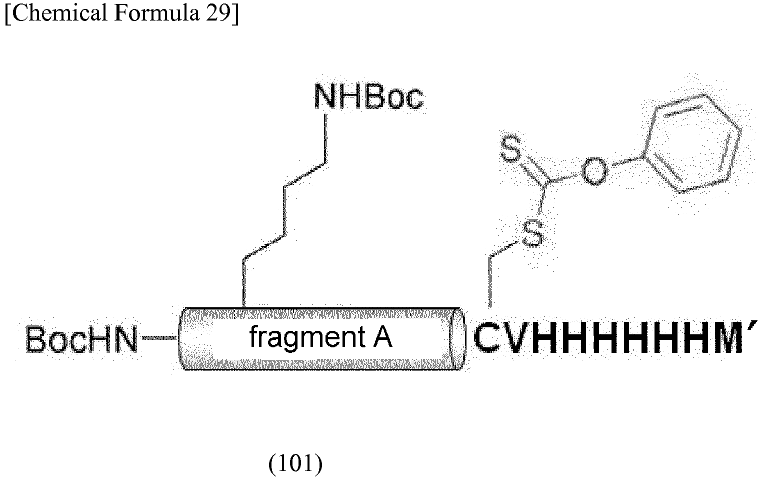

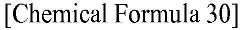

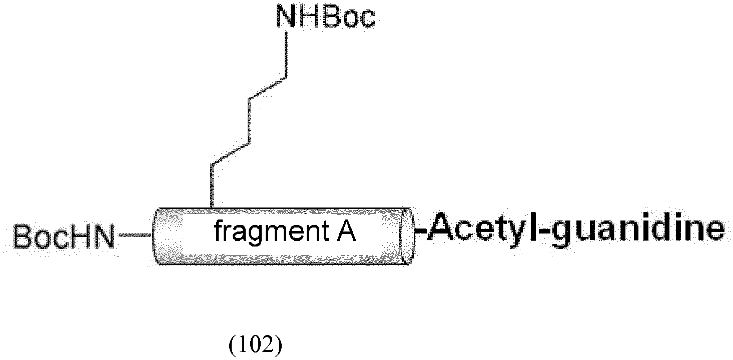

### 7. Synthesis of polypeptide fragment B (amino acids 28 - 43)

The extension of polypeptide fragment B (compound 2) was synthesized employing solid phase synthesis by a general Fmoc or Boc method.

94.34 mg (50 µmol) of Boc-Leu-PAM resin was placed in a solid phase synthesis tube, and sufficiently washed with distilled DMF and distilled DCM and then dried. The amino group of the amino acid employed for condensation was those protected by a Boc group. Moreover, the reaction was performed in the solid phase synthesis tube unless otherwise particularly described.

This was washed well with DMF and DCM, and then 10% sulfuric acid/dioxane solution (1.0 ml) was added to the resin and stirred for 30 minutes to deprotect the Boc group, and the resin was washed with DCM and DMF.

Then, 5 equivalents of S-trityol-3-mercaptopropionic acid (87.1 mg, 250 µmol) as the linker, 10 equivalents of DIPEA (87.3 µl, 500 µmol), and 5 equivalents of HBTU (94.8 mg, 250 µmol) were dissolved in DMF (1.0 ml), placed in a tube for solid phase synthesis filled with resin prepared as above, and stirred at room temperature for 0.5 hours. After stirring, the resin was washed several times with DCM and DMF, and subsequently washed well with DCM and DMF. Then, TFA was added to the resin and stirred for 30 minutes to deprotect the trityl group, and the resin was washed with DCM and DMF.

Next, after washing the resin with DMF, 5 equivalents of the first residue Boc-Tyr(Br-Z)-COOH (123.6 mg, 250 µmol), 5 equivalents of HBTU (94.8 mg, 50 µmol), and 10 equivalents of DIPEA (87.3 µl, 500 µmol) were mixed in a DMF solvent (1.0 ml), this was added to the resin and stirred at room temperature for 0.5 hours to yield an intermediate (compound 14).

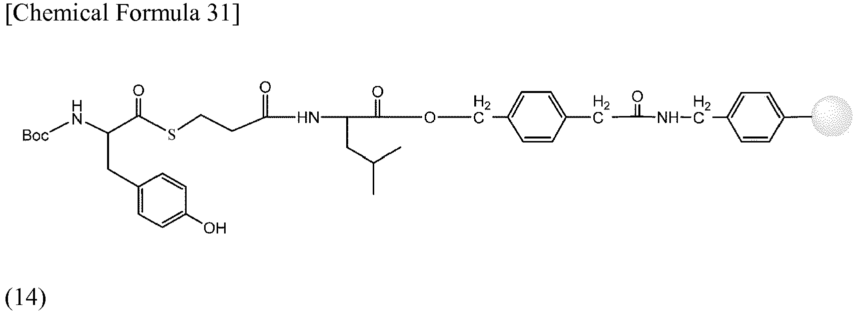

After condensation, the resin was washed with DMF. It was then confirmed with a Kayser test that the amino acid was condensed on the resin, and the Boc group was deprotected sequentially as with the Boc-Leu-PAM-Resin. Similar condensation was performed with residue 2 and the subsequent amino acids. All of these were completed with one cycle of each condensation step (single coupling) until residue 16.

Those protected with a thiazolidine-type was used for Cys corresponding to amino acid 28 in the amino acid sequence of IL-13.

The resin after the completion of the 28 amino acids condensation was washed well with DMF and DCM, 1 ml of TFA:DMS:m-cresol:EDT:TfOH = 5:3:0.8:0.2:1 cocktail was added to the resin, and stirred on ice for 1 hour to deprotect the side chain from the resin. This was washed well with diethyl ether and dried.

### 8. Cleaving out from resin

In order to cleave out the peptide prepared in the above 7. from the resin, 1 ml of TFA:Thioanisol:EDT:TfOH = 8:0.8:0.2:0.8 cocktail was added to the resin on ice and stirred for 1 hour. After precipitation with diethyl ether, this was dried at room temperature, and then the sample was analyzed with HPLC (column: Synmetory300™ C4, 3.5 µm, 4.6 x 150 mm, flow rate: 1.0 ml/min, 18 minutes of a linear gradient of 0.09% TFA containing 18% - 54% CH₃CN). At a retention time of 13 minutes, a peptide thioester form (compound 103) having a sequence of amino acids 28 - 43 in the amino acid sequence of IL13 that was cleaved out as a thioester form due to the linker being added was obtained. This peptide thioester (compound 103) (SEQ ID NO. 10) was purified with HPLC, and the fractionated solution was lyophilized to yield 5 mg. IL13 (28 - 43) thioester form (compound 103); ESI-MS: m/z C₈₅H₁₃₀N₂₀O₂₅S₄; calculated: [M+H]⁺ 1959.8, [M+2H]²⁺ 980.4, found: 1960.8, 981.0

### 9. Synthesis of polypeptide fragment C of glycosylated portion (amino acids 44 - 55)

A solid phase synthesis method by a general Fmoc method was employed for the extension of polypeptide fragment C (compound 3).

1.67 mg (50 µmol) of 4-(4-hydroxymethyl-3-methoxyphenoxy)-butyric acid (HMPB)-poly(ethylene glycol)-poly(dimethylacrylamide) copolymer (PEGA) resin was placed in a solid phase synthesis tube, and sufficiently washed with distilled DMF and distilled DCM and then dried. The amino group of the amino acid employed for condensation was those protected by an Fmoc group. Moreover, the reaction was performed in the solid phase synthesis tube unless otherwise particularly described.

5 equivalents of Fmoc-Gly-OH (74.3 mg, 250 µmol) and 3.75 equivalents of N-metylimidazole (14.9 µl, 187.5 µmol) were dissolved in DCM (1.0 ml), placed in a tube for solid phase synthesis filled with resin prepared as above, and stirred at room temperature for 2 hours. After stirring, the resin was washed several times with DCM:MeOH:DIPEA = 17:2:1, and subsequently washed well with DCM and DMF. Then, 20% piperidine/DMF solution (1.0 ml) was added to the resin and stirred for 30 minutes to deprotect the Fmoc group, and the resin was washed with DMF.

Next, after washing the resin with DMF, 5 equivalents of residue 2 Fmoc-Ser(tButyl)-COOH (95.9 mg, 50 µmol), 5 equivalents of HOBt·H₂O (33.8 mg, 50 µmol), and 5 equivalents of DIPCDI (38.5 µl, 50 µmol) were mixed in a DMF solvent (1.0 ml), and this was added to the resin and stirred at room temperature for 1 hour. After condensation, the resin was washed with DMF. It was then confirmed with a Kayser test that the amino acid was condensed on the resin, and the Fmoc group was deprotected in a manner similar to residue 1. Condensation was similarly performed with residue 3. Next, Fmoc-Asn-asialo sugar chain represented by the following formula (20) was condensed as residue 4.

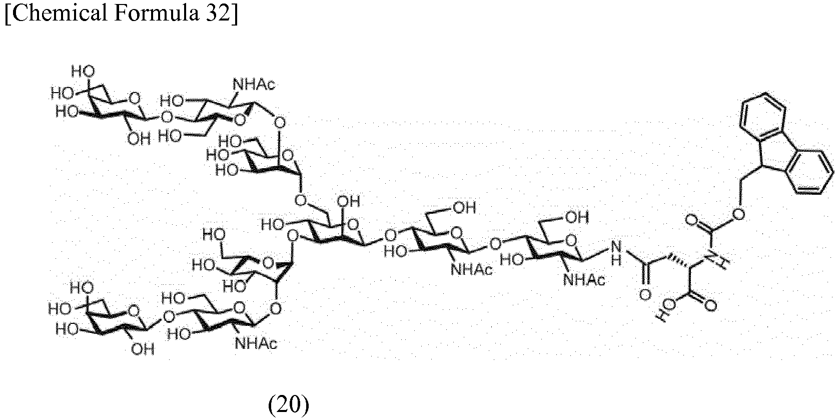

In other words, after deprotecting the Fmoc group of valine at residue 3, the resin was washed with DMF, and then added in DMSO:DMF solvent 4:1 (2.5 ml) containing Fmoc-Asn-asialo sugar chain (from Otsuka Chemical Co., Ltd., 198 mg, 100 µmol), 3 equivalents of DEPBT (45 mg, 150 µmol), and 2 equivalents of DIPEA (18.5 µl, 100 µmol) to introduce a glycosylated asparagine at residue 4. Then, the condensation of the amino acid was performed in a manner similar to the amino acids at residues 1 - 3, except that that the amount of DMF was adjusted so that the concentrations of the Fmoc-protected amino acids employed in the reaction solution were all approximately 50 mM. After adding the glycosylated asparagine, the Fmoc-protected amino acids were completed with one cycle of each condensation step (single coupling).

The Cys positioned at the N-terminal (Cys corresponding to amino acid 45 in the amino acid sequence of IL-13) employed was those protected with a thiazolidine-type.

The resin after the completion of condensation was washed well with DMF and DCM, 2 ml of AcOH:DCM:MeOH = 5:4:1 cocktail was added to the resin and stirred for 3 hours, and then a sugar chain-containing polypeptide fragment having the amino acid side chain protected (compound 104) (SEQ ID NO. 11) was cleaved out from the resin. This was concentrated under reduced pressure to dryness at room temperature, and acetic acid was azeotropically removed with benzene. To a portion of this, 95% TFA, 2.5% triisopropylsilane (TIS), and 2.5% H₂O were added to remove the protecting group of the amino acid side chain other than the Cys corresponding to amino acid 44 in the amino acid sequence of IL-13, and after concentrating under reduced pressure at room temperature, the purity was identified with high performance liquid chromatography (HPLC). At a retention time of 11 minutes, the sugar chain-containing polypeptide fragment C of interest (compound 105) (SEQ ID NO. 12) was confirmed from the mass spectrometry result.
Compound 105: ESI-MS: m/z C₁₁₂H₁₈₇N₁₇O₆₃S; calculated: [M+2H]²⁺ 1406.1, [M+3H]³⁺ 937.7, found: 1406.8, 938.3

### 10. Thioesterification of sugar chain-containing polypeptide fragment C

The sugar chain-containing polypeptide fragment having the amino acid side chain protected obtained in the above 9. (compound 104) was sugject to azeotropic treatment with benzene, and then to dry in a desiccator. 10.1 mg (3.3 µmol) of this polypeptide fragment (compound 104) was dissolved in 0.4 ml of DMF, 4Å dried molecular sieves (6 mg) and 30 equivalents of benzyl thiol (11.6 µl, 99 µmol) were added, stirred at -20°C for 1 hour, 5 equivalents of PyBOP (8.58 mg, 16.5 µmol) and 5 equivalents of DIPEA (2.9 µl, 16.5 µmol) were added, and reacted for 2 hours to perform thioesterification of the C-terminal. After completion of the reaction, the reaction solution was added dropwise to 6 ml of diethyl ether, and a white precipitate was collected by centrifugal separation. The precipitate was washed several times with diethyl ether, dried, and a sugar chain-containing polypeptide thioester (compound 106) (SEQ ID NO. 13) was obtained as a white solid. To this solid, 1 ml of TFA:H₂O:TIS:EDT = 90:5:2.5:2.5 cocktail was added and stirred for 3 hours to remove the protecting group of the peptide side chain. After concentrating under reduced pressure at room temperature, the sample was analyzed with HPLC (column: Synmetory300™ C4, 3.5 µm, 4.6 x 150 mm, flow rate: 1.0 ml/min, 15 minutes of a linear gradient of 0.09% TFA containing 18% - 54% CH₃CN). At a retention time of 9.5 minutes, a sugar chain-containing polypeptide thioester having amino acids 44 - 55 in the amino acid sequence of IL13 (compound 107) (SEQ ID NO. 14) was obtained. This sugar chain-containing polypeptide thioester (compound 107) was purified with HPLC, and the fractionated solution was lyophilized to yield 7.2 mg.
IL13 (44 - 55) thioester form (compound 107); ESI-MS: m/z C₁₁₉H₁₉₃N₁₇O₆₂S₂; calculated: [M+2H]²⁺ 1459.1, [M+3H]³⁺973.1, found: 1459.7, 973.5

### 11. Linking of sugar chain-containing polypeptide fragment C and polypeptide fragment D

The thioesterified sugar chain-containing polypeptide fragment obtained in the above 10. (compound 107) (1.2 mg, 425 nmol) and the non-glycosylated portion which is polypeptide fragment D obtained in the above 5. (compound 4) (2.3 mg, 355 nmol) were added to 180 µl of buffer solution (6 M guanidine hydrochloride, 0.2 M PBS, 20 mM TCEP (tris(2-carboxyethyl)phosphine), and 0.04 M MPAA (4-mercaptophenylacetic acid), pH 6.8), and reacted at room temperature for 3 hours to yield a sugar chain-containing polypeptide fragment of sugar chain-containing polypeptide fragment C and polypeptide fragment D linked together (compound 108) (SEQ ID NO. 15). After the completion of NCL, methoxyamine hydrochloride (3.0 mg, 35.9 µmol) was directly added to the reaction solution, pH was adjusted to 4.0, the N-terminal thiazolidine was converted into cysteine. After 12 hours, the reaction mixture was analyzed with HPLC, and a corresponding peak was observed. Moreover, the compound at this peak was purified, and mass spectrometry confirmed that 2.3 mg of a sugar chain-containing polypeptide fragment (compound 109) (SEQ ID NO. 16) was obtained.
Compound 109: ESI-MS: m/z C₄₀₂H₆₅₄N₁₀₂O₁₄₁S₃; calculated: [M+4H]⁴⁺ 2316.7, [M+5H]⁵⁺ 1853.5, [M+6H]⁶⁺ 1544.8, [M+7H]⁷⁺ 1324.2, [M+8H]⁸⁺ 1158.8, [M+9H]⁹⁺ 1030.2, found: 2317.7, 1854.5, 1545.7, 1325.2, 1030.9

### 12. Linking of polypeptide fragment B and sugar chain-containing polypeptide fragment C·D

The sugar chain-containing polypeptide fragment obtained in the above 11. (compound 109) (1.3 mg, 140 nmol) and the thioesterified polypeptide fragment B obtained in the above 8. (compound 103) (0.40 mg, 209 nmol) were added to 70 µl of buffer solution (6 M guanidine hydrochloride, 0.2 M PBS, 40 mM TCEP (tris(2-carboxyethylethyl)phosphine), and 0.02 M MPAA (4-mercaptophenylacetic acid), pH 7.1), and reacted at room temperature for 8 hours to yield a sugar chain-containing polypeptide fragment of polypeptide fragment B and sugar chain-containing polypeptide fragment C·D linked together (compound 110) (SEQ ID NO. 17). After the completion of NCL, methoxyamine hydrochloride (1.2 mg, 14 µmol) was directly added to the reaction solution, pH was adjusted to 4.0, the N-terminal thiazolidine was converted into cysteine. After 12 hours, the reaction mixture was analyzed with HPLC, and a corresponding peak was observed. Moreover, the compound at this peak was purified, and mass spectrometry confirmed that 0.6 mg of a sugar chain-containing polypeptide fragment (compound 111) (SEQ ID NO. 18) was obtained.
Compound 111: ESI-MS: m/z C₄₇₇H₇₆₇N₁₂₁O₁₆₃S₆; calculated: [M+5H]⁵⁺ 2199.1, [M+6H]⁶⁺ 1832.7, [M+7H]⁷⁺ 1571.1, [M+8H]⁸⁺ 1374.8, [M+9H]⁹⁺ 1222.2, [M+10H]¹⁰⁺ 1100.0, [M+11H]¹¹⁺ 1000.1, found: 2200.4, 1834.1, 1572.3, 1375.9, 1223.3, 1100.9, 1000.9

### 13. Linking of polypeptide fragment A and sugar chain-containing polypeptide fragment B·C·D

The sugar chain-containing polypeptide fragment (compound 111) (0.6 mg, 54 nmol) obtained in the above 12. and the guanindine-derivatized polypeptide fragment (compound 102) (0.2 mg, 62 nmol) obtained in the above 6. were added to 50 µl of buffer solution (6 M guanidine hydrochloride, 0.2 M PBS, 20 mM TCEP (tris(2-carboxyethylethyl)phosphine), and 0.1 M MPAA (4-mercaptophenylacetic acid), pH 7.2), and reaction was allowed at room temperature for 24 hours. After 24 hours, the reaction mixture was analyzed with HPLC, and a corresponding peak was observed. The compound at this peak was purified, and mass spectrometry confirmed that 0.1 mg of a sugar chain-containing polypeptide (compound 112) (SEQ ID NO. 19) was obtained.
Compound 112: ESI-MS: m/z C₆₁₇H₁₀₀₀N₁₅₆O₂₀₇S₆; calculated: [M+8H]⁸⁺ 1763.4, [M+9H]⁹⁺ 1567.6, [M+10H]¹⁰⁺ 1410.9, [M+11H]¹¹⁺ 1282.7, [M+12H]¹²⁺ 1175.9, [M+13H]¹³⁺ 1085.5, [M+14H]¹⁴⁺ 1008.1, found: 1764.3, 1568.5, 1411.8, 1283.6, 1176.8, 1086.3, 1009.0

### 14. Removal of Boc group

0.1 mg of the 112-residue sugar chain-containing polypeptide obtained in the above 13. (compound 112) was dissolved in 200 µL 5% aqueous trifluoroacetic acid, and reaction was allowed under room temperature for 1 hour. The solution after the reaction was concentrated by blowing argon gas, then diluted with 200 µL of buffer solution (6 M guanidine hydrochloride and 0.2 M PBS), and the solution obtained was purified with HPLC. The main peak was collected, and 0.1 mg of the interleukin-13 derivative having uniform sugar chain of interest (compound 113) (SEQ ID NO. 1) was obtained.

### 15. Optimization of intervening sequence

In order to optimize W in the intervening sequence (-Cys-W-(His)n-Z-Met-), the isolated yields of the guanidide form produced in the guanididation of a peptide thionoformate having a His tag at the C-terminal (reaction formula is shown below) was compared. 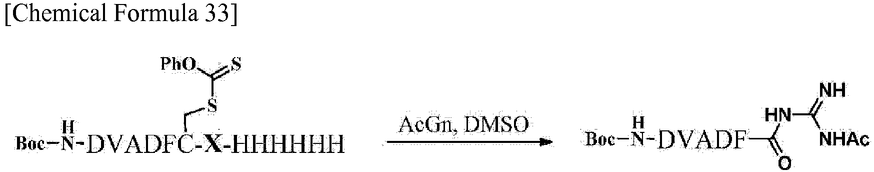 X = G, V, I, P, F, W, L
(D indicates Asp, V indicates Val, A indicates Ala, F indicates Phe, C indicates Cys, and X indicates any one amino acid of Gly, Val, Ile, Pro, Phe, Trp, and Leu.)

Six peptide thionoformates having different amino acids for Xaa in Boc-Asp-Val-Ala-Asp-Phe-Cys(C(S)OPh)-Xaa-His-His-His-His-His-His-OH (Xaa = Gly, Val, Ile, Phe, Pro, and Trp) (SEQ ID NO. 20) were used for the guanididation reaction. To the peptide thionoformate/DMSO solution comprising each peptide thionoformate (2 mM) was added a same volume of 1-acetylguanidine/DMSO solution (1 M), and stirred by a vortex mixer. This was left in a warm water bath at 37°C for 1 day, and then precipitated with diethyl ether and washed. This was purified with a semi-preparative HPLC to yield the compound of interest (SEQ ID NO. 21). The isolated yield of each peptide is described in Table 1. ESI MS calcd [M+H]⁺ 748.3 found. 748.3.

From the results shown in Table 1, an extremely large amount of the guanidino form could be obtained when Val, Trp, or Ile are inserted on the N-terminal side of the His tag. In particular, Val and Ile could suppress the production of a byproduct.

**[Table 1]**

| Sequence | **X**=amino acid | Guanidino form | Byproduct |
|---|---|---|---|
| | G | 16% | 24% |
| | V | 56% | Trace * |
| DVADFC**X** | I | 46% | Trace* |
| -HHHHHH | F | 36% | 17% |
| | P | 26% | Not detected |
| | W | 53% | 14% |

| | | | |
|---|---|---|---|
| *:Detected by mass spectroscopy | | | |

## Claims

1. A method for efficiently manufacturing a first polypeptide fragment having cysteine at the N-terminal and a second polypeptide fragment having the C-terminal modified which are suitable for the NCL method, comprising:
(1) A step of reacting a polypeptide having the following structure:
(N-terminal) second polypeptide fragment-Cys-W-(His)n-Z-Met-first polypeptide fragment (C-terminal)
[wherein n means an integer from 0 - 10, Cys is cysteine, W means any 1, 2, or 3 amino acids, Z means any 0, 1, or 2 amino acids, His means histidine, Met means methionine, and
the N-terminal of the first polypeptide fragment is cysteine]
with CNBr to obtain the following polypeptide fragments:
(A) a first polypeptide fragment having cysteine at the N-terminal and
(B) a third polypeptide fragment having the following structure:
(N-terminal) second polypeptide fragment-Cys-W-(His)n-Z-Met' (C-terminal)
[wherein Met' means a Met derivative];
(2) A step of reacting said third polypeptide fragment with a compound represented by the following formula (I): 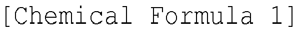 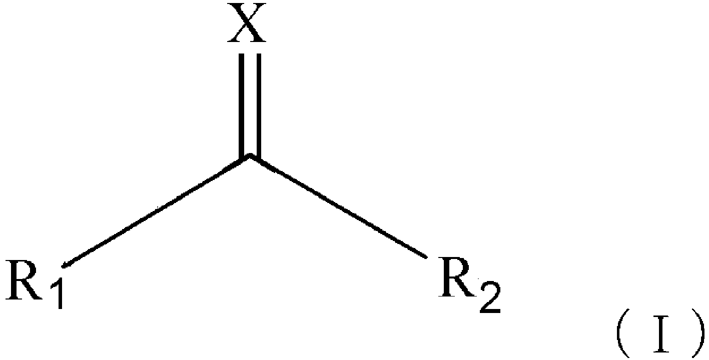 [wherein
X is a sulfur atom or an oxygen atom, and
R₁ and R₂ are leaving groups]
and subsequently, in an organic solvent, reacting with a compound represented by the following formula (II): 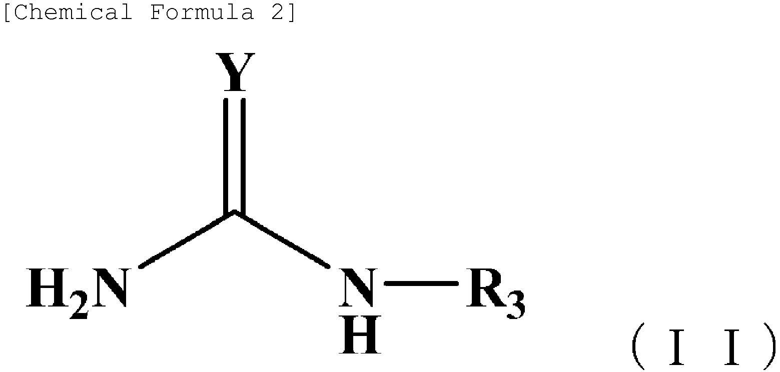 [wherein
Y is an oxygen atom, a sulfur atom, or =NH, and
R₃ is a hydrogen atom, an acyl group, or an alkoxycarbonyl group]
to obtain a second polypeptide fragment having the C-terminal modified having the following structure:
(N-terminal) second polypeptide fragment-C(=O)-NH-C(=Y)NHR₃ (C-terminal).

2. A manufacturing method according to claim 1, further comprising:
a step of reacting the second polypeptide fragment having the C-terminal modified obtained in said step (2) with further a thiol represented by the following formula
R₄-SH
[wherein R₄ is any one group selected from the group consisting of a substituted or unsubstituted benzyl group, a substituted or unsubstituted aryl group, and a substituted or unsubstituted alkyl group]
and exchanging the -NH-C(=Y)NHR₃ group at the C-terminal with the thiol group to obtain a second polypeptide fragment having the C-terminal modified having the following structure:
(N-terminal) second polypeptide fragment-C(=O)-SR₄ (C-terminal).

3. A manufacturing method according to claim 1 or 2, **characterized in that** X in the compound represented by said formula (I) is a sulfur atom.

4. A manufacturing method according to any one of claims 1 to 3, **characterized in that** R₁ in the compound represented by said formula (I) is an -O-C₆ aryl group.

5. A manufacturing method according to any one of claims 1 to 4, **characterized in that** R₂ in the compound represented by said formula (I) is a halogen atom or a substituted or unsubstituted -S-C₆₋₁₀ aryl group.

6. A manufacturing method according to claims 1 to 5, **characterized in that** said polypeptide having the following structure:
(N-terminal) second polypeptide fragment-Cys-W-(His)n-Z-Met-first polypeptide fragment (C-terminal)
is a recombinant polypeptide fragment expressed by a cell.

7. A manufacturing method according to claim 6, **characterized in that** said cell is *E. coli.*

8. A manufacturing method according to any one of claims 1 to 7, **characterized in that** W is one amino acid, and is any one amino acid selected from the group consisting of Val, Ile, Leu, and Trp.

9. A manufacturing method according to any one of claims 1 to 8, **characterized in that** n is an integer from 6 - 10.

10. A method for manufacturing a glycosylated polypeptide, comprising:
(1) A step of classifying and designing the peptide sequence of the desired glycosylated polypeptide to be manufactured into at least:
• a sugar chain-containing polypeptide fragment consisting of a polypeptide comprising a glycosylated amino acid,
• a second polypeptide fragment present on the N-terminal side of the sugar chain-containing polypeptide fragment and consisting of a polypeptide comprising the N-terminal side of the desired glycosylated peptide,
• a first polypeptide fragment present on the C-terminal side of the sugar chain-containing polypeptide fragment and consisting of a polypeptide comprising the C-terminal side of the desired glycosylated peptide,
• when it may be present, a polypeptide fragment between the sugar chain-containing polypeptide fragment and the second polypeptide fragment, and
• when it may be present, a polypeptide fragment between the sugar chain-containing polypeptide fragment and the first polypeptide fragment,
wherein the N-terminal of the first polypeptide fragment is designed to be a cysteine;
(2) A step of obtaining a polypeptide having said structure by employing an expression vector comprising a nucleotide sequence encoding a polypeptide having the following structure:
(N-terminal) second polypeptide fragment-Cys-W-(His)n-Z-Met-first polypeptide fragment (C-terminal) [wherein n means an integer from 0 - 10, Cys is cysteine, W means any 1, 2, or 3 amino acids, Z means any 0, 1, or 2 amino acids, His means histidine, and Met means methionine,
and the N-terminal of the first polypeptide fragment is cysteine]
and allowing expression from *E. coli;*
(3) A step of reacting the polypeptide obtained in step (2) with CNBr to obtain the following polypeptide fragments:
(A) a first polypeptide fragment having cysteine at the N-terminal and
(B) a third polypeptide fragment having the following structure:
(N-terminal) second polypeptide fragment-Cys-W-(His)n-Z-Met' (C-terminal)
(wherein Met' means a Met derivative);
(4) A step of reacting said third polypeptide fragment with a compound represented by the following formula (I): 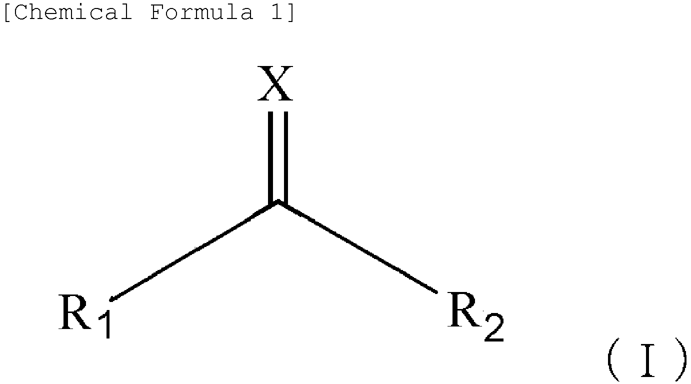 [wherein X is a sulfur atom or an oxygen atom, and R₁ and R₂ are leaving groups]
and subsequently, in an organic solvent, reacting with a compound represented by the following formula (II): 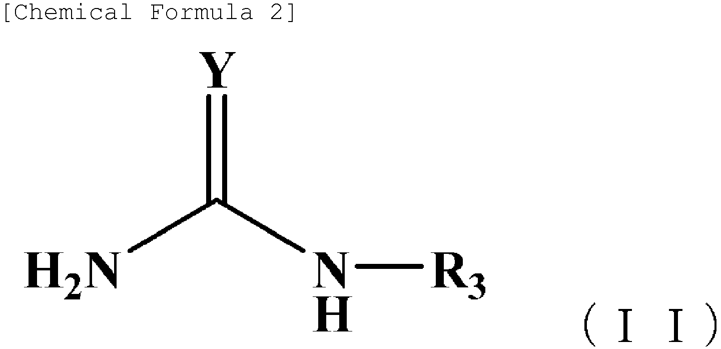 [wherein
Y is an oxygen atom, a sulfur atom, or a NH group, and
R₃ is a hydrogen atom, an acyl group, or an alkoxycarbonyl group]
to obtain a second polypeptide fragment having the C-terminal modified having the following structure: (N-terminal) second polypeptide fragment-C(=O)-NH-C(=Y)NHR₃ (C-terminal) ;
(5) Arbitrarily, a step of reacting the second polypeptide fragment having the C-terminal modified obtained in said step (4) with further a thiol represented by the following formula
R₄-SH
[wherein R₄ is any one group selected from the group consisting of a substituted or unsubstituted benzyl group, a substituted or unsubstituted aryl group, and a substituted or unsubstituted alkyl group]
and exchanging the -NH-C(=Y)NHR₃ group at the C-terminal with the thiol group to obtain a second polypeptide fragment having the C-terminal modified having the following structure:
(N-terminal) second polypeptide fragment-C(=O)-SR₄ (C-terminal); and
(6) A step of linking:
• said sugar chain-containing polypeptide fragment,
• when it may be present, said polypeptide fragment between the sugar chain-containing polypeptide fragment and the second polypeptide fragment, and
• when it may be present, said polypeptide fragment between the sugar chain-containing polypeptide fragment and the first polypeptide fragment
separately prepared by chemical synthesis with:
the first polypeptide fragment having cysteine at the N-terminal obtained in step (3) and
the second polypeptide fragment having the C-terminal modified obtained in step (4) or (5)
in an order that will give the desired glycosylated polypeptide by ligation.

11. A manufacturing method according to claim 10, **characterized in that** X in the compound represented by said formula (I) is a sulfur atom.

12. A manufacturing method according to claim 10 or 11, **characterized in that** R₁ in the compound represented by said formula (I) is an -O-C₆ aryl group.

13. A manufacturing method according to any one of claims 10 to 12, **characterized in that** R₂ in the compound represented by said formula (I) is a halogen atom or a substituted or unsubstituted -S-C₆₋₁₀ aryl group.

14. A manufacturing method according to any one of claims 10 to 13, **characterized in that** W is one amino acid, and is any one amino acid selected from the group consisting of Val, Ile, Leu, and Trp.

15. A manufacturing method according to any one of claims 10 to 14, **characterized in that** n is an integer from 6 - 10.

## Patentansprüche

1. Verfahren zum effizienten Herstellen eines ersten Polypeptidfragments, das Cystein am N-Terminus hat, und eines zweiten Polypeptidfragments, bei dem der C-Terminus modifiziert ist, die für das NCL-Verfahren geeignet sind, umfassend:
(1) Einen Schritt des Umsetzens eines Polypeptids, das die folgende Struktur hat:
(N-Terminus) zweites Polypeptidfragment-Cys-W-(His)n-Z-Met-erstes Polypeptidfragment (C-Terminus)
[wobei n eine Ganzzahl von 0 - 10 bedeutet, Cys Cystein ist, W eine beliebige aus 1, 2 oder 3 Aminosäuren bedeutet, Z eine beliebige aus 0, 1 oder 2 Aminosäuren bedeutet, His Histidin bedeutet, Met Methionin bedeutet und der N-Terminus des ersten Polypeptidfragments Cystein ist]
mit CNBr, um die folgenden Polypeptidfragmente zu erhalten:
(A) ein erstes Polypeptidfragment, das Cystein am N-Terminus hat und
(B) ein drittes Polypeptidfragment, das die folgende Struktur hat:
(N-Terminus) zweites Polypeptidfragment-Cys-W-(His)n-Z- Met' (C-Terminus) [wobei Met' ein Met-Derivat bedeutet];
(2) Einen Schritt des Umsetzens des dritten Polypeptidfragments mit einer Verbindung, die durch die folgende Formel (I) dargestellt ist: 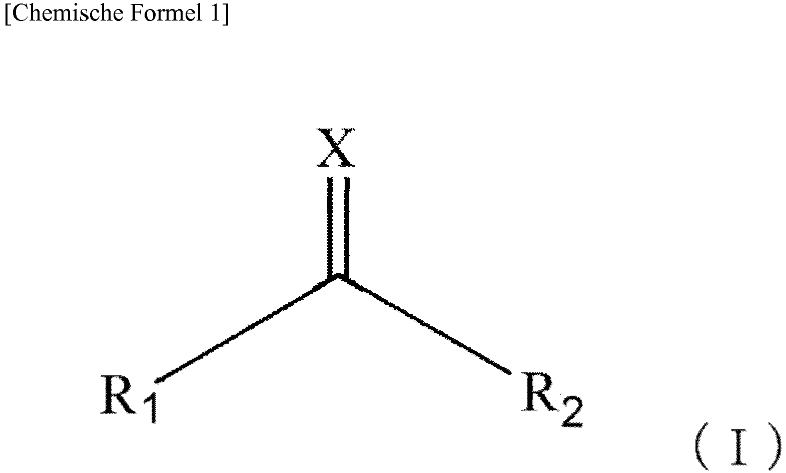 [wobei
X ein Schwefelatom oder ein Sauerstoffatom ist und
R₁ und R₂ Abgangsgruppen sind]
und anschließend, in einem organischen Lösungsmittel, Umsetzen mit einer Verbindung, die durch die folgende Formel (II) dargestellt ist: 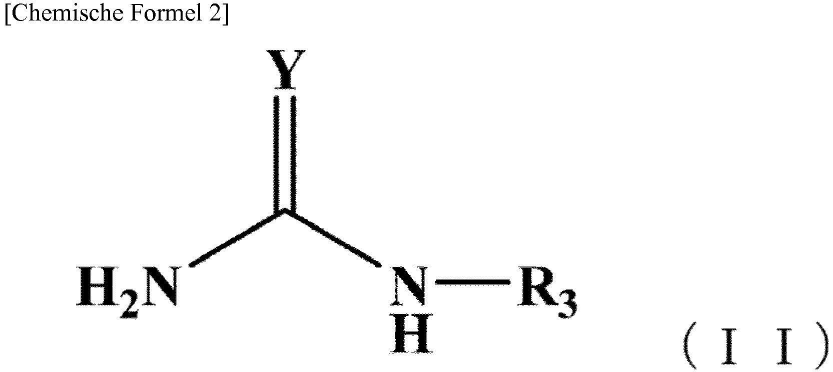 [wobei
Y ein Sauerstoffatom, ein Schwefelatom oder =NH ist und
R₃ ein Wasserstoffatom, eine Acylgruppe oder eine Alkoxycarbonylgruppe ist],
um ein zweites Polypeptidfragment zu erhalten, bei dem der C-Terminus modifiziert ist, das die folgende Struktur hat:
(N-Terminus) zweites Polypeptidfragment-C(=O)-NH-C(=Y)NHR₃ (C-Terminus).

2. Herstellungsverfahren nach Anspruch 1, ferner umfassend:
einen Schritt des Umsetzens des ersten Polypeptidfragments, bei dem der C-Terminus modifiziert ist, das in dem Schritt (2) erhalten wurde, mit ferner einem Thiol, das durch die folgende Formel dargestellt ist:
R₄-SH
[wobei R₄ eine beliebige Gruppe ist, ausgewählt aus der Gruppe bestehend aus einer substituierten oder unsubstituierten Benzylgruppe, einer substituierten oder unsubstituierten Arylgruppe und einer substituierten oder unsubstituierten Alkylgruppe]
und Austauschen der -NH-C(=Y)NHR₃-Gruppe am C-Terminus mit der Thiolgruppe, um ein zweites Polypeptidfragment zu erhalten, bei dem der C-Terminus modifiziert ist, das die folgende Struktur hat: (N-Terminus) zweites Polypeptidfragment-C(=O)-SR₄ (C- Terminus).

3. Herstellungsverfahren nach Anspruch 1 oder 2, **dadurch gekennzeichnet, dass** X in der durch die Formel (I) dargestellten Verbindung ein Schwefelatom ist.

4. Herstellungsverfahren nach einem der Ansprüche 1 bis 3, **dadurch gekennzeichnet, dass** R₁ in der durch die Formel (I) dargestellten Verbindung eine -O-C₆-Arylgruppe ist.

5. Herstellungsverfahren nach einem der Ansprüche 1 bis 4, **dadurch gekennzeichnet, dass** R₂ in der durch die Formel (I) dargestellten Verbindung ein Halogenatom oder eine substituierte oder unsubstituierte S-C₆₋₁₀-Arylgruppe ist.

6. Herstellungsverfahren nach einem der Ansprüche 1 bis 5, **dadurch gekennzeichnet, dass** das Polypeptid, das die folgende Struktur hat:
(N-Terminus) zweites Polypeptidfragment-Cys-W-(His)n-Z- Met-erstes Polypeptidfragment (C-Terminus)
ein rekombinantes Polypeptidfragment ist, das von einer Zelle exprimiert wird.

7. Herstellungsverfahren nach Anspruch 6, **dadurch gekennzeichnet, dass** die Zelle *E. coli* ist.

8. Herstellungsverfahren nach einem der Ansprüche 1 bis 7, **dadurch gekennzeichnet, dass** W eine Aminosäure ist und eine beliebige Aminosäure ist, ausgewählt aus der Gruppe bestehend aus Val, Ile, Leu und Trp.

9. Herstellungsverfahren nach einem der Ansprüche 1 bis 8, **dadurch gekennzeichnet, dass** n eine Ganzzahl von 6 - 10 ist.

10. Verfahren zum Herstellen eines glykosylierten Polypeptids, umfassend:
(1) Einen Schritt des Klassifizierens und Konstruierens der Peptidsequenz des gewünschten herzustellenden glykosylierten Polypeptids in mindestens:
• ein Zuckerkette enthaltendes Polypeptidfragment, das aus einem Polypeptid besteht, das eine glykosylierte Aminosäure umfasst,
• ein zweites Polypeptidfragment, das auf der N-terminalen Seite des Zuckerkette enthaltenden Polypeptidfragments vorhanden ist und aus einem Polypeptid besteht, das die N-terminale Seite des gewünschten glykosylierten Peptids umfasst,
• ein erstes Polypeptidfragment, das auf der C-terminalen Seite des Zuckerkette enthaltenden Polypeptidfragments vorhanden ist und aus einem Polypeptid besteht, das die C-terminale Seite des gewünschten glykosylierten Peptids umfasst,
• wenn es vorhanden sein sollte, ein Polypeptidfragment zwischen dem Zuckerkette enthaltenden Polypeptidfragment und dem zweiten Polypeptidfragment und
• wenn es vorhanden sein sollte, ein Polypeptidfragment zwischen dem Zuckerkette enthaltenden Polypeptidfragment und dem ersten Polypeptidfragment,
wobei der N-Terminus des ersten Polypeptidfragments konstruiert ist, um ein Cystein zu sein;
(2) Einen Schritt des Erhaltens eines Polypeptids, das die Struktur hat, durch Einsetzen eines Expressionsvektors, umfassend eine Nukleotidsequenz, die für ein Polypeptid kodiert, das die folgende Struktur hat:
(N-Terminus) zweites Polypeptidfragment-Cys-W-(His)n-Z-Met-erstes Polypeptidfragment (C-Terminus)
[wobei n eine Ganzzahl von 0 - 10 bedeutet, Cys Cystein ist, W eine beliebige aus 1, 2 oder 3 Aminosäuren bedeutet, Z eine beliebige aus 0, 1 oder 2 Aminosäuren bedeutet, His Histidin bedeutet und Met Methionin bedeutet und der N-Terminus des ersten Polypeptidfragments Cystein ist]
und Erlauben der Expression von *E. coli;*
(3) Einen Schritt des Umsetzens des Polypeptids, das in Schritt (2) erhalten wurde, mit CNBr, um die folgenden Peptidfragmente zu erhalten:
(A) ein erstes Polypeptidfragment, das Cystein am N-Terminus hat, und
(B) ein drittes Polypeptidfragment, das die folgende Struktur hat:
(N-Terminus) zweites Polypeptidfragment-Cys-W-(His)n-Z- Met' (C-Terminus) (wobei Met' ein Met-Derivat bedeutet);
(4) Einen Schritt des Umsetzens des dritten Polypeptidfragments mit einer Verbindung, die durch die folgende Formel (I) dargestellt ist: 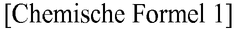 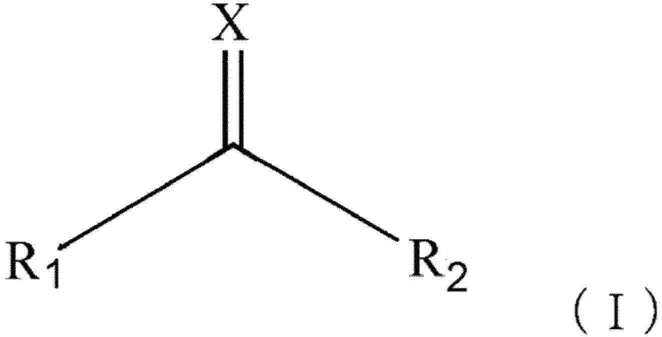 [wobei X ein Schwefelatom oder ein Sauerstoffatom ist und R₁ und R₂ Abgangsgruppen sind]
und anschließend, in einem organischen Lösungsmittel, Umsetzen mit einer Verbindung, die durch die folgende Formel (II) dargestellt ist: 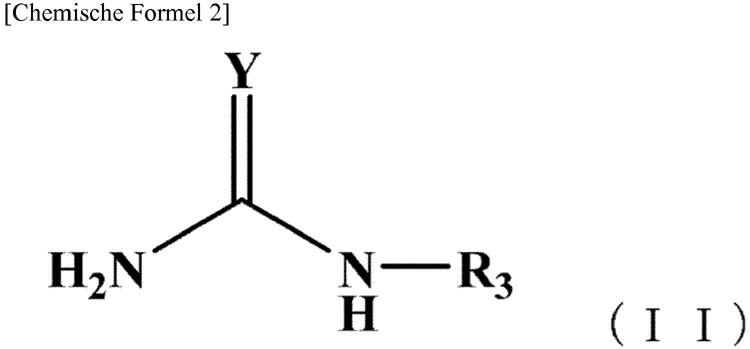 [wobei
Y ein Sauerstoffatom, ein Schwefelatom oder eine NH-Gruppe ist und
R₃ ein Wasserstoffatom, eine Acylgruppe oder eine Alkoxycarbonylgruppe ist],
um ein zweites Polypeptidfragment zu erhalten, bei dem der C-Terminus modifiziert ist, das die folgende Struktur hat:
(N-Terminus) zweites Polypeptidfragment - C(=O)-NH-C(=Y)NHR₃ (C-Terminus);
(5) Beliebig einen Schritt des Umsetzens des zweiten Polypeptidfragments, bei dem der C-Terminus modifiziert ist, das in dem Schritt (4) erhalten wurde, mit ferner einem Thiol, das durch die folgende Formel dargestellt ist:
R₄-SH
[wobei R₄ eine beliebige Gruppe ist, ausgewählt aus der Gruppe bestehend aus einer substituierten oder unsubstituierten Benzylgruppe, einer substituierten oder unsubstituierten Arylgruppe und einer substituierten oder unsubstituierten Alkylgruppe] und Austauschen der -NH-C(=Y)NHR₃-Gruppe beim C-Terminus mit der Thiolgruppe, um ein zweites Polypeptidfragment zu erhalten, bei dem der C-Terminus modifiziert ist, das die folgende Struktur hat:
(N-Terminus) zweites Polypeptidfragment-C(=O)-SR₄ (C-Terminus); und
(6) Einen Schritt des Verbindens von:
• dem Zuckerkette enthaltenden Polypeptidfragment,
• wenn es vorhanden sein sollte, dem Polypeptidfragment zwischen dem Zuckerkette enthaltenden Polypeptidfragment und dem zweiten Polypeptidfragment und
• wenn es vorhanden sein sollte, dem Polypeptidfragment zwischen dem
Zuckerkette enthaltenden Polypeptidfragment und dem ersten Polypeptidfragment, die separat durch chemische Synthese mit Folgenden zubereitet wurden:
dem ersten Polypeptidfragment, das Cystein am N-Terminus hat, das in Schritt (3) erhalten wurde, und
dem zweiten Polypeptidfragment, bei dem der C-Terminus modifiziert wurde, das in Schritt (4) oder (5) erhalten wurde,
in einer Reihenfolge, die das gewünschte glykosylierte Polypeptid durch Ligation ergeben wird.

11. Herstellungsverfahren nach Anspruch 10, **dadurch gekennzeichnet, dass** X in der durch die Formel (I) dargestellten Verbindung ein Schwefelatom ist.

12. Herstellungsverfahren nach Anspruch 10 oder 11, **dadurch gekennzeichnet, dass** R₁ in der durch die Formel (I) dargestellten Verbindung eine -O-C₆-Arylgruppe ist.

13. Herstellungsverfahren nach einem der Ansprüche 10 bis 12, **dadurch gekennzeichnet, dass** R₂ in der durch die Formel (I) dargestellten Verbindung ein Halogenatom oder eine substituierte oder unsubstituierte -S-C₆₋₁₀-Arylgruppe ist.

14. Herstellungsverfahren nach einem der Ansprüche 10 bis 13, **dadurch gekennzeichnet, dass** W eine Aminosäure ist und eine beliebige Aminosäure ist, ausgewählt aus der Gruppe bestehend aus Val, Ile, Leu und Trp.

15. Herstellungsverfahren nach einem der Ansprüche 10 bis 14, **dadurch gekennzeichnet, dass** n eine Ganzzahl von 6 - 10 ist.

## Revendications

1. Procédé destiné à fabriquer de manière efficace un premier fragment de polypeptide comportant la cystéine au niveau de la terminaison N et un deuxième fragment de polypeptide comportant la terminaison C modifiée qui sont appropriés le procédé NCL, comprenant :
(1) une étape de mise en réaction d'un polypeptide de structure suivante :
(terminaison N) deuxième fragment de polypeptide fragment-Cys-W-(His)n-Z-Met-premier fragment de polypeptide (terminaison C)
[où n représente un nombre entier allant de 0 à 10, Cys représente la cystéine, W représente un quelconque élément parmi 1, 2 ou 3 acides aminés, Z représente un quelconque élément parmi 0, 1 ou 2 acides aminés, His représente l'histidine, Met représente la méthionine et la terminaison N du premier fragment de polypeptide est la cystéine]
avec CNBr pour obtenir les fragments de polypeptide suivants :
(A) un premier fragment de polypeptide comportant la cystéine au niveau de la terminaison N et
(B) un troisième fragment de polypeptide de structure suivante :
(terminaison N) deuxième fragment de polypeptide-Cys-W-(His)n-Z-Met' (terminaison C)
[Met' représentant un dérivé de Met] ;
(2) une étape de mise en réaction dudit troisième fragment de polypeptide avec un composé représenté par la formule suivante (I) : 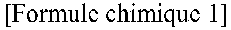 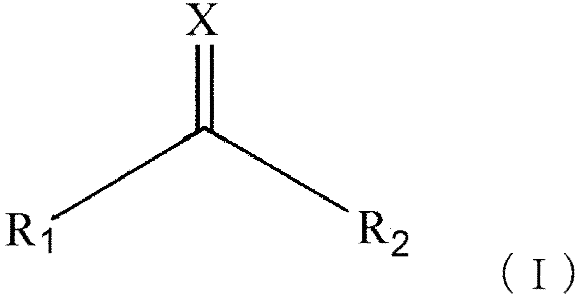 [où
X est un atome de soufre ou un atome d'oxygène, et
R₁ et R₂ représentent des groupes labiles]
et ensuite, dans un solvant organique, la mise en réaction avec un composé représenté par la formule suivante (II) : 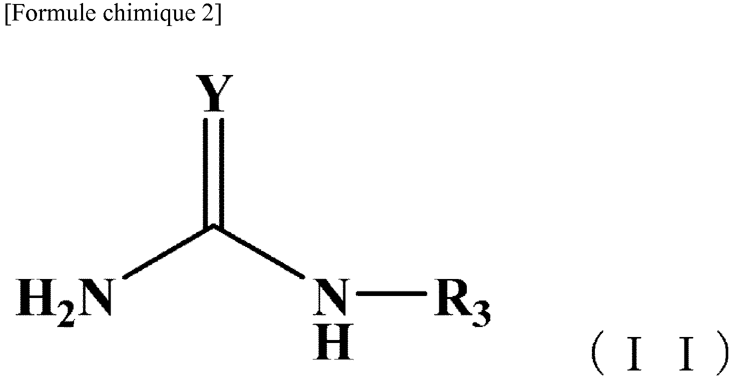 [où
Y est un atome d'oxygène, un atome de soufre ou =NH, et
R₃ est un atome d'hydrogène, un groupe acyle ou un groupe alcoxycarbonyle]
pour obtenir un deuxième fragment de polypeptide comportant la terminaison C modifiée de structure suivante :
(terminaison N) deuxième fragment de polypeptide-C(=O)-NH-C(=Y)NHR₃ (terminaison C).

2. Procédé de fabrication selon la revendication 1, comprenant en outre :
une étape de mise en réaction du deuxième fragment de polypeptide comportant la terminaison C modifiée obtenue dans ladite étape (2) avec en outre un thiol représenté par la formule suivante
R₄-SH
[R₄ étant un quelconque groupe choisi dans le groupe constitué par un groupe benzyle substitué ou non substitué, un groupe aryle substitué ou non substitué et un groupe alkyle substitué ou non substitué]
et un échange du groupe -NH-C(=Y)NHR₃ au niveau de la terminaison C avec le groupe thiol pour obtenir un deuxième fragment de polypeptide comportant la terminaison C modifiée de structure suivante :
(terminaison N) deuxième fragment de polypeptide-C(=O)-SR₄ (terminaison C).

3. Procédé de fabrication selon la revendication 1 ou 2, **caractérisé en ce que** X dans le composé représenté par ladite formule (I) est un atome de soufre.

4. Procédé de fabrication selon l'une quelconque des revendications 1 à 3, **caractérisé en ce que** R₁ dans le composé représenté par ladite formule (I) est un groupe aryle -O-C₆.

5. Procédé de fabrication selon l'une quelconque des revendications 1 à 4, **caractérisé en ce que** R₂ dans le composé représenté par ladite formule (I) est un atome d'halogène ou un groupe aryle -S-C₆₋₁₀ substitué ou non substitué.

6. Procédé de fabrication selon les revendications 1 à 5, **caractérisé en ce que** ledit polypeptide de structure suivante :
(terminaison N) deuxième fragment de polypeptide-Cys-W-(His)n-Z-Met-premier fragment de polypeptide (terminaison C)
est un fragment de polypeptide recombinant exprimé par une cellule.

7. Procédé de fabrication selon la revendication 6, **caractérisé en ce que** ladite cellule est *E. coli.*

8. Procédé de fabrication selon l'une quelconque des revendications 1 à 7, **caractérisé en ce que** W est un acide aminé, et est un quelconque acide aminé choisi dans le groupe constitué par Val, Ile, Leu et Trp.

9. Procédé de fabrication selon l'une quelconque des revendications 1 à 8, **caractérisé en ce que** n est un nombre entier allant de 6 à 10.

10. Procédé de fabrication d'un polypeptide glycosylé, comprenant :
(1) une étape de classification et de conception de la séquence peptidique du polypeptide glycosylé désiré devant être fabriqué en au moins :
- un fragment de polypeptide contenant une chaîne de sucre constitué d'un polypeptide comprenant un acide aminé glycosylé,
- un deuxième fragment de polypeptide présent sur le côté de la terminaison N du fragment de polypeptide contenant une chaîne de sucre et constitué d'un polypeptide comprenant le côté de la terminaison N du peptide glycosylé désiré,
- un premier fragment de polypeptide présent sur le côté de la terminaison C du fragment de polypeptide contenant une chaîne de sucre et constitué d'un polypeptide comprenant le côté de la terminaison C du peptide glycosylé désiré,
- lorsqu'il peut être présent, un fragment de polypeptide entre le fragment de polypeptide contenant une chaîne de sucre et le deuxième fragment de polypeptide, et
- lorsqu'il peut être présent, un fragment de polypeptide entre le fragment de polypeptide contenant une chaîne de sucre et le premier fragment de polypeptide,
la terminaison N du premier fragment de polypeptide étant conçue pour être une cystéine ;
(2) une étape d'obtention d'un polypeptide présentant ladite structure au moyen d'un vecteur d'expression comprenant une séquence nucléotidique codant pour un polypeptide de structure suivante :
(terminaison N) deuxième fragment de polypeptide-Cys-W-(His)n-Z-Met-premier fragment de polypeptide (terminaison C)
[n représentant un nombre entier allant de 0 à 10, Cys représentant la cystéine, W représentant un quelconque élément parmi 1, 2 ou 3 acides aminés, Z représentant un quelconque élément parmi 0, 1 ou 2 acides aminés, His représentant l'histidine et Met représentant la méthionine et la terminaison N du premier fragment de polypeptide étant la cystéine]
et consistant à permettre l'expression à partir d'*E. coli* ;
(3) une étape de mise en réaction du polypeptide obtenu dans l'étape (2) avec CNBr pour obtenir les fragments de polypeptide suivants :
(A) un premier fragment de polypeptide comportant la cystéine au niveau de la terminaison N et
(B) un troisième fragment de polypeptide de structure suivante :
(terminaison N) deuxième fragment de polypeptide-Cys-W-(His)n-Z-Met' (terminaison C) (où Met' représente un dérivé de Met) ;
(4) une étape de mise en réaction dudit troisième fragment de polypeptide avec un composé représenté par la formule suivante (I) : 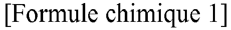 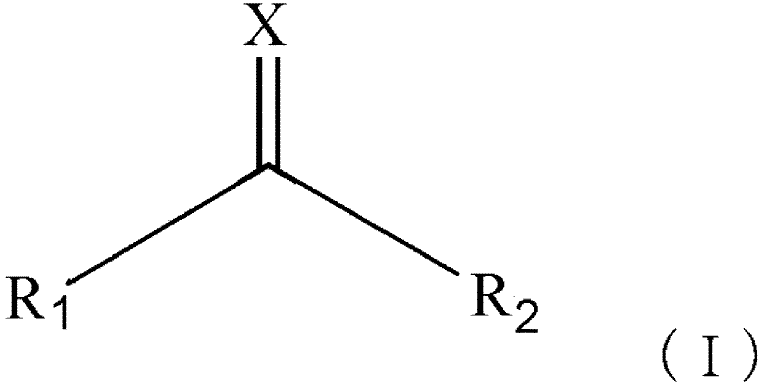 [où X est un atome de soufre ou un atome d'oxygène et R₁ et R₂ sont des groupes labiles] et ensuite, dans un solvant organique, la mise en réaction avec un composé représenté par la formule suivante (II) : 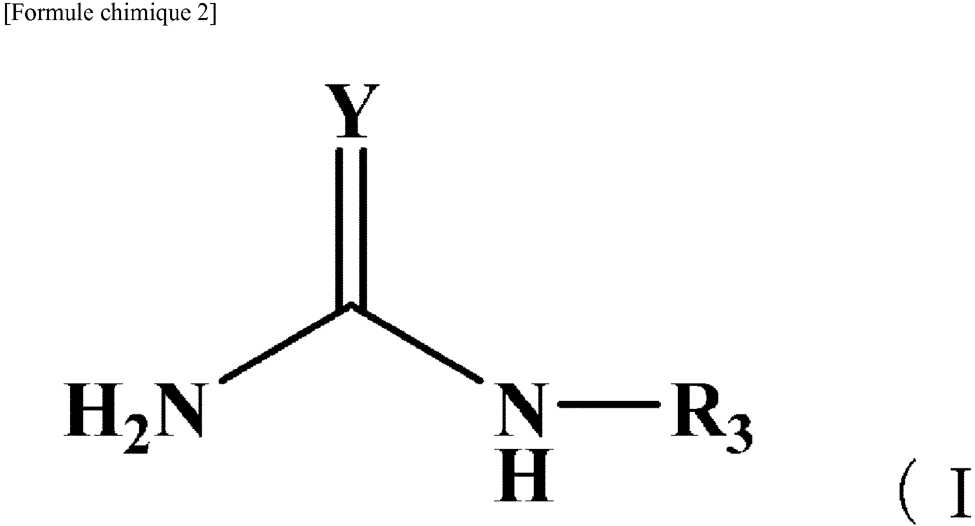 [où
Y est un atome d'oxygène, un atome de soufre ou un groupe NH, et
R₃ est un atome d'hydrogène, un groupe acyle ou un groupe alcoxycarbonyle] pour obtenir un deuxième fragment de polypeptide comportant la terminaison C modifiée de structure suivante :
(terminaison N) deuxième fragment de polypeptide-C(=O)-NH-C(=Y)NHR₃ (terminaison C);
(5) arbitrairement, une étape de mise en réaction du deuxième fragment de polypeptide comportant la terminaison C modifiée dans ladite étape (4) avec en outre un thiol représenté par la formule suivante
R₄-SH
[où R₄ représente un quelconque groupe choisi dans le groupe constitué par un groupe benzyle substitué ou non substitué, un groupe aryle substitué ou non substitué et un groupe alkyle substitué ou non substitué]
et l'échange du groupe -NH-C(=Y)NHR₃ au niveau de la terminaison C avec le groupe thiol pour obtenir un deuxième fragment de polypeptide comportant la terminaison C modifiée de structure suivante :
(terminaison N) deuxième fragment de polypeptide-C(=O)-SR₄ (terminaison C) ; et
(6) une étape de liaison :
- dudit fragment de polypeptide contenant une chaîne de sucre,
- lorsqu'il peut être présent, dudit fragment de polypeptide entre le fragment de polypeptide contenant une chaîne de sucre et le deuxième fragment de polypeptide, et
- lorsqu'il peut être présent, dudit fragment de polypeptide entre le fragment de polypeptide contenant une chaîne de sucre et le premier fragment de polypeptide préparé séparément par une synthèse chimique avec :
le premier fragment de polypeptide comportant la cystéine au niveau de la terminaison N obtenue dans l'étape (3) et
le deuxième fragment de polypeptide comportant la terminaison C modifiée obtenue dans l'étape (4) ou (5) dans un ordre qui donnera le polypeptide glycosylé désiré par ligature.

11. Procédé de fabrication selon la revendication 10, **caractérisé en ce que** X dans le composé représenté par ladite formule (I) est un atome de soufre.

12. Procédé de fabrication selon la revendication 10 ou 11, **caractérisé en ce que** R₁ dans le composé représenté par ladite formule (I) est un groupe aryle -O-C₆.

13. Procédé de fabrication selon l'une quelconque des revendications 10 à 12, **caractérisé en ce que** R₂ dans le composé représenté par ladite formule (I) est un atome d'halogène ou un groupe aryle -S-C₆₋₁₀ substitué ou non substitué.

14. Procédé de fabrication selon l'une quelconque des revendications 10 à 13, **caractérisé en ce que** W est un acide aminé, et est un quelconque acide aminé choisi dans le groupe constitué par Val, Ile, Leu et Trp.

15. Procédé de fabrication selon l'une quelconque des revendications 10 à 14, **caractérisé en ce que** n est un nombre entier allant de 6 à 10.
